# EUROPÄISCHE PATENTSCHRIFT

(11) **EP 1 692 129 B1**
(45) Veröffentlichungstag und Bekanntmachung des Hinweises auf die Patenterteilung: **20.08.2008**
(21) Anmeldenummer: 04803759.2
(22) Anmeldetag: 10.12.2004
(51) Int. Cl.: C07D 403/12, C07D 401/12, C07D 223/16, A61K 31/55, A61P 13/12, A61P 25/28

(54) **KETOLACTAM-VERBINDUNGEN UND IHRE VERWENDUNG**
KETO LACTAM COMPOUNDS AND USE THEREOF
COMPOSES DE CETOLACTAME ET SON UTILISATION

(30) Priorität: 11.12.2003 DE 10358004
(43) Veröffentlichungstag der Anmeldung: 23.08.2006
(73) Patentinhaber: Abbott GmbH & Co. KG, 65205 Wiesbaden (DE)
(72) Erfinder: LUBISCH, Wilfried, 69118 Heidelberg (DE); HAUPT, Andreas, 68723 Schwetzingen (DE); BRAJE, Wilfried, 31737 Rinteln (DE); GENESTE, Hervé, 67141 Neuhofen (DE)
(74) Vertreter: Reitstötter - Kinzebach
(86) Internationale Anmeldenummer: PCT/EP2004/014118
(87) Internationale Veröffentlichungsnummer: WO 2005/056546

(56) Entgegenhaltungen:
- WO-A-00/76981
- WO-A-97/25324
- ORJALES A ET AL: "New 3-benzisothiazolyl and 3-benzisoxazolylpiperazine derivatives with atypical antipsychotic binding profile" EUROPEAN JOURNAL OF MEDICINAL CHEMISTRY, EDITIONS SCIENTIFIQUE ELSEVIER, PARIS, FR, Bd. 37, Nr. 9, September 2002 (2002-09), Seiten 721-730, XP004383155 ISSN: 0223-5234
- HACKLING A E ET AL: "DOPAMINE D3 RECEPTOR LIGANDS WITH ANTAGONIST PROPERTIES" CHEMBIOCHEM - A EUROPEAN JOURNAL OF CHEMICAL BIOLOGY, WILEY VCH, WEINHEIM, DE, Bd. 3, Nr. 10, 4. Oktober 2002 (2002-10-04), Seiten 946-961, XP001154341 ISSN: 1439-4227
- LEOPOLDO M ET AL: "Structure-affinity relationship study on N-[4-(4-arylpiperazin-1-yl)b utyl]arylcarboxamides as potent and selective dopamine D3 receptor ligands" JOURNAL OF MEDICINAL CHEMISTRY, AMERICAN CHEMICAL SOCIETY. WASHINGTON, US, Bd. 45, Nr. 26, 22. November 2002 (2002-11-22), Seiten 5727-5735, XP002256410 ISSN: 0022-2623

## Beschreibung

Die vorliegende Erfindung betrifft neue Ketolactam-Verbindungen, hydrierte Derivate und Tautomere davon. Diese Verbindungen besitzen wertvolle therapeutische Eigenschaften und sind insbesondere zur Behandlung von Erkrankungen geeignet, die auf die Modulation des Dopamin-D₃-Rezeptors ansprechen.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin. Es liegen gesicherte Erkenntnisse über die Anwesenheit von Dopamin und dessen physiologische Funktion als Neurotransmitter vor. Störungen im dopaminergen Transmittersystem resultieren in Erkrankungen des zentralen Nervensystems, zu denen z. B. Schizophrenie, Depression oder Parkinson-Krankheit zählen. Eine mögliche Behandlung dieser und anderer Erkrankungen basiert auf der Gabe von Substanzen, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren. In jüngerer Zeit wurde ein dritter Subtyp gefunden, nämlich der D₃-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514, J.N. Joyce, Pharmacology and Therapeutics 2001, 90, S. 231-59'The Dopamine D₃ Receptor as a Therapeutic Target for Antipsychotic and Antiparkinsonian Drugs").

Mittlerweile teilt man die Dopamin-Rezeptoren in zwei Familien ein: Einerseits die D₂-Gruppe, bestehend aus D₂-, D₃- und D₄-Rezeptoren, andererseits die D₁-Gruppe bestehend aus D₁- und D₅-Rezeptoren. Während D₁- und D₂-Rezeptoren weit verbreitet sind, scheinen D₃-Rezeptoren hingegen regioselektiv exprimiert zu werden. So findet man diese Rezeptoren vorzugsweise im limbischen System, den Projektionsbereichen des mesolimbischen Dopaminsystems, vor allem im Nucleus accumbens, aber auch in anderen Bereichen, wie der Amygdala. Wegen dieser vergleichsweise regioselektiven Expression gelten D₃-Rezeptoren als nebenwirkungsarmes Target, und es wird angenommen, dass ein selektiver D₃-Ligand wohl die Eigenschaften bekannter Antipsychotika, nicht aber ihre Dopamin-D₂-Rezeptor-vermittelten neurologischen Nebenwirkungen haben sollte (P. Sokoloff et al., Localization and Function of the D3 Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature 347, 146 (1990)).

Verbindungen mit Dopamin-D₃-Rezeptoraffinität wurden verschiedentlich im Stand der Technik beschrieben, z.B. in WO 96/02519, WO 96/02520, WO 96/02249, WO 96/02246, WO 97/25324, WO 00/42036. DE 10131543 und WO 99/02503. Diese Verbindungen besitzen teilweise hohe Affinitäten zum Dopamin-D₃-Rezeptor. Sie werden daher zur Behandlung von Erkrankungen des zentralen Nervensystems vorgeschlagen.

Es besteht jedoch grundsätzlich ein Bedarf, weitere Verbindungen mit Dopamin-D₃-Rezeptoraffinität bereitzustellen, sei es, um das pharmakologische Bindungsprofil zu verbessern, oder weil die Verbindungen Standes der Technik unerwünschte Nebenwirkungen hervorrufen, eine schlechte Cerebralverfügbarkeit aufweisen oder nur eine geringe Bioverfügbarkeit besitzen. Der Erfindung liegt daher die Aufgabe zu Grunde, weitere Verbindungen zur Verfügung zu stellen, die als selektive Dopamin-D₃-Rezeptor-Liganden wirken.

Diese Aufgabe wird gelöst durch die Derivate von Ketolactamen, welche der allgemeinen Formel I 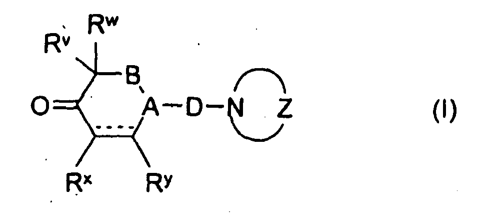 worin 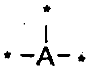 für eine Gruppe der Formel 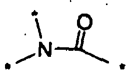 steht, worin D an das Stickstoffatom gebunden ist und worin das Kohlenstoffatom an die Variable B gebunden ist und * die Bindungsstellen angibt;
- -B-: für eine Bindung oder für 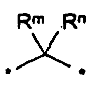 steht, worin R^{m} und Rⁿ unabhängig voneinander ausgewählt sind unter Wasserstoff, Halogen, gegebenenfalls substituiertem C₁-C₆-Alkyl, C₃-C₆-Cycloalkyl, C₂-C₆-Alkenyl, C₂-C₆-Alkinyl, C₁-C₆-Alkoxy, C₃-C₆-Cycloalkyloxy, C₃-C₆-Cycloalkyl-C₁-C₄alkyloxy und gegebenenfalls substituiertem Phenyl, und * die Bindungsstellen angibt;
- ···: für eine Einfachbindung oder eine Doppelbindung steht;
- R^{v}, R^{w}: unabhängig voneinander Wasserstoff, Halogen, gegebenenfalls substituiertes C₁-C₆-Alkyl, C₁-C₆-Alkoxy, C₂-C₆-Alkenyl, C₂-C₆-Alkinyl, C₃-C₆-Cycloalkyloxy, C₃-C₆-Cycloalkyl-C₁-C₄-alkyloxy oder C₃-C₆-Cycloalkyl bedeuten;
- R^{x}, R^{y}: gemeinsam mit den C-Atomen, an die sie gebunden sind, für einen kondensierten Phenylring oder ein kondensierten 5- oder 6-gliedrigen, aromatischen Heterocyclus stehen, der 1, 2, 3 oder 4 Heteroatome aufweist, die ausgewählt sind unter N, O und S, wobei der kondensierte Phenylring sowie der kondensierte aromatische Heterocyclus 1, 2 oder 3 Substituenten aufweisen können, die ausgewählt sind unter C₁-C₄-Alkyl, C₁-C₄-Hydroxyalkyl, C₁-C₄-Alkoxy-C₁-C₄-alkyl, CN, OR¹, NA²R³, NO₂, SR⁴, SO₂R⁴, SO₂NR²R³, CONR²R³, COOR⁵, COR⁶, C₁-C₂-Fluoralkyl, C₁C₂-Fluoralkoxy, C₂-C₆-Alkenyl, C₂-C₆-Alkinyl, C₂-C₆-Alkenyloxy, C₂-C₆-Alkinyloxy, C₃-C₆-Cyctoalkyl und Halogen, worin
R¹, R², R³, R⁴, R⁵ und R⁶ unabhängig voneinander für H, gegebenenfalls substituiertes C₁-C₆-Alkyl oder gegebenenfalls substituiertes Phenyl, stehen, wobei R³ auch eine Gruppe COR⁷ bedeuten kann, wobei R⁷ für Wasserstoff, gegebenenfalls substituiertes C₁-C₄-Alkyl oder gegebenenfalls substituiertes Phenyl stehen, wobei R² mit R³ auch gemeinsam einen 5- oder 6-gliedrigen, gesättigten oder ungesättigten Carbocyclus bilden kann, der ein Heteroatom, ausgewählt unter O, S und NR⁸ als Ringglied aufweisen kann, wobei R⁸ für Wasserstoff oder C₁-C₄-Alkyl steht,
- D: eine Gruppe (CH₂)ₖ oder eine Gruppe C(O)(CH₂)ₗ bedeutet, worin k für 3, 4, 5 oder 6 und I für 2, 3, 4 oder 5 stehen; 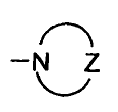 für einen Rest der Formel 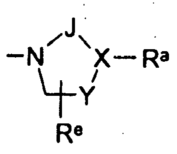 steht, worin
J für CH₂, CH₂-CH₂ oder CH₂-CH₂-CH₂ steht;
X für CH oder N und
Y für CH₂, CH₂-CH₂ oder CH₂-CH₂-CH₂ stehen oder Y-X gemeinsam CH=C oder CH₂-CH=C bedeuten;
R^{e} Wasserstoff oder C₁-C₄-Alkyl bedeutet, und
R^{a} für eine Gruppe E-Ar steht, worin E für eine Bindung oder für lineares oder verzweigtes Alkylen mit 1 bis 4 C-Atomen und insbesondere für (CH₂)ₚ steht, worin p für 0, 1, 2, 3 oder 4 steht, und Ar ausgewählt ist unter Phenyl, Naphthyl und 5- oder 6-gliederigem Heteroaryl, das ein, zwei oder drei Heteroatome ausgewählt unter S, O und N als Ringglieder aufweist und das gegebenenfalls 1, 2 oder 3 Substituenten R^{b} aufweisen kann, wobei R^{b} unabhängig voneinander ausgewählt sind unter gegebenenfalls substituiertem C₁-C₆-Alkyl, C₂-C₆-Alkenyl, C₂-C₆-Alkinyl, C₃-C₆-Cycloalkyl, C₄-C₁₀-Bicycloalkyl und C₈-C₁₀-Tricycloalkyl, wobei die drei letztgenannten Gruppen gegebenenfalls durch Halogen oder C₁-C₄-Alkyl substituiert sein können, Halogen, CN, OR¹, NR²R³, NO₂, SR⁴, SO₂R⁵, CONR²R³, SO₂NR²R³, COOR⁵, COR⁶, O-COR⁶, 5- oder 6-gliedriges Heterocyclyl mit 1, 2 oder 3 Heteroatomen, ausgewählt unter O, S und N, und Phenyl, wobei Phenyl und Heterocyclyl in den zwei zuletzt genannten Substituenten R^{b} gegebenenfalls ein oder zwei Substituenten tragen, die unabhängig voneinander ausgewählt sind unter C₁-C₄-Alkyl, C₁-C₄-Alkoxy, NR²R³, CN, C₁-C₂-Fluoralkyl und Halogen, und wobei 2 an benachbarte C-Atome des aromatischen Rests gebundene Substituenten R^{b} gemeinsam für C₃-oder C₄-Alkylen stehen können oder gemeinsam mit den C-Atomen, an die sie gebunden sind, für einen ankondensierten, ungesättigten 5 oder 6-gliedrigen Carbocyclus oder für einen 5- oder 6-gliedrigen Heterocyclus mit 1 oder 2 Stickstoffatomen als Ringglieder stehen können;
sowie
die physiologisch akzeptablen Säureadditionssalze dieser Verbindungen sowie die Tautomere der Formel I' 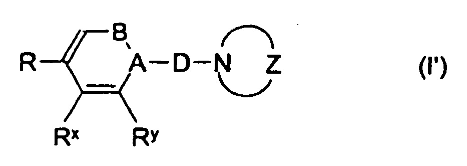 worin R für Halogen, eine Gruppe O-R', worin R¹ die zuvor genannten Bedeutungen aufweist, oder für eine Gruppe O-C(O)R⁹ steht, wobei R⁹ für Wasserstoff, gegebenenfalls substituiertes C₁-C₆-Alkyl, Benzyl oder Phenyl steht, wobei die zwei letztgenannten Reste gegebenenfalls durch ein oder zwei Reste substituiert ist, die unabhängig voneinander ausgewählt sind unter C₁-C₄-Alkyl, OH, C₁-C₄-Alkoxy, NR²R³, CN, C₁-C₂-Fluoralkyl oder Halogen, und die physiologisch akzeptablen Säureadditionssalze der Tautomere I',
wobei der Ausdruck "gegebenenfalls substituiertes Alkyl" in den Restedefinitionen von B, R^{v}, R^{w}, R¹, R², R³, R⁴, R⁵, R⁶, R⁷, R⁹, R^{b} und in der Restedefinition von "gegebenenfalls substituiertem Phenyl" für Alkyl steht, das teilweise oder vollständig durch Halogen substituiert sein kann und das einen oder mehrere von Halogen verschiedene Substituenten tragen kann, die ausgewählt sind unter CN, C₃-C₇-Cycloalkyl, C₃-C₇-Heterocycloalkyl, gegebenenfalls substituiertem Phenyl, OR¹¹, COOR¹¹, NR¹²R¹³, SO₂NR¹²R¹³, CONR¹²R¹³, O-CONR¹²R¹³, S-R¹⁴, SOR¹⁵, SO₂R¹⁵, OCOR¹⁶ und COR¹⁶, wobei R¹¹ die für R' angegebene Bedeutung, R¹² die für R² angegebene Bedeutung, R¹³ die für R³ angegebene Bedeutung, R¹⁴ die für R⁴ angegebene Bedeutung, R¹⁵ die für R⁵ angegebene Bedeutung und R¹⁶ die für R⁶ angegebene Bedeutung aufweisen, und
der Ausdruck "gegebenenfalls substituiertes Phenyl" in den Restedefinitionen von B, R¹, R², R³, R⁴, R⁵, R⁶, R⁷ und in der Restedefinition von "gegebenenfalls substituiertem Alkyl" für Phenyl steht, das gegebenenfalls einen oder mehrere Substituenten aufweist, die ausgewählt sind unter Halogen, Nitro, Cyano, gegebenenfalls substituiertem C₁-C₄-Alkyl, C₂-C₆-Alkenyl, C₂-C₆-Alkinyl, C₃-C₆-Cycloalkyl, C₁-C₄-Halogenalkyl, C₁-C₄-Alkoxy, OR²¹, COOR²¹, NR²²R²³, SO₂NR²²R²³, CONR²²R²³, O-CONR²²R²³, S-R²⁴, SOR²⁵, SO₂R²⁵, OCOR²⁶ und COR²⁶, wobei R²¹ die für R¹ angegebene Bedeutung, R²² die für R² angegebene Bedeutung, R²³ die für R³ angegebene Bedeutung, R²⁴ die für R⁴ angegebene Bedeutung, R²⁵ die für R⁵ angegebene Bedeutung und R²⁶ die für R⁶ angegebene Bedeutung aufweisen.

Gegenstand der vorliegenden Erfindung sind daher die Verbindungen der allgemeinen Formel I, die Tautomere der Formel I' sowie die physiologisch verträglichen Säureadditionssalze der Verbindungen I und ihrer Tautomere I'.

Gegenstand der vorliegenden Erfindung ist auch die Verwendung Verbindungen der allgemeinen Formel I, der Tautomere der Formel I' sowie der physiologisch verträglichen Säureadditionssalze der Verbindungen I und ihrer Tautomere I' zur Herstellung eines pharmazeutischen Mittels zur Behandlung von Erkrankungen, die auf die Beeinflussung durch Dopamin-D₃-Rezeptorantagonisten bzw. -agonisten ansprechen.

Zu den Erkrankungen, die auf die Beeinflussung durch Dopamin-D₃-Rezeptor-antagonisten bzw. -agonisten ansprechen zählen insbesondere Störungen und Erkrankungen des zentralen Nervensystems, insbesondere affektiven Störungen, neurotische Störungen, Belastungs- und somatoformen Störungen und Psychosen, speziell Schizophrenie und Depression und weiterhin Nierenfunktionsstörungen, insbesondere Nierenfunktionsstörungen, die durch Diabetes-Mellitus hervorgerufen werden (siehe WO 00/67847).

Erfindungsgemäß verwendet man zur Behandlung der vorstehend genannten Indikationen wenigstens eine Verbindung der allgemeinen Formel I, ein Tautomere der Formel I' oder ein physiologisch verträglichen Säureadditionssalze einer Verbindung I oder eines Tautomere I'. Sofern die Verbindungen der Formel I oder ihre Tautomere I' ein oder mehrere Asymmetriezentren aufweisen, können auch Enantiomerengemische, insbesondere Racemate, Diastereomerengemische, Tautomerengemische, vorzugsweise jedoch die jeweiligen im Wesentlichen reinen Enantiomere, Diastereomere und Tautomere eingesetzt werden.

Ebenfalls brauchbar sind physiologisch verträgliche Salze der Verbindungen der Formel I sowie der Tautomere I', vor allem Säureadditionssalze mit physiologisch verträglichen Säuren. Als physiologisch verträgliche organische und anorganische Säuren kommen beispielsweise Salzsäure, Bromwasserstoffsäure, Phosphorsäure, Schwefelsäure, C₁-C₄-Alkylsulfonsäuren wie Methansulfonsäure, aromatische Sulfansäuren wie Benzolsulfonsäure und Toluolsulfonsäure, Oxalsäure, Maleinsäure, Fumarsäure, Milchsäure, Weinsäure, Adipinsäure und Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Halogen steht hier und im folgenden für Fluor, Chlor, Brom oder Iod.

Cₙ-Cₘ-Alkyl (auch in Resten wie Alkoxy, Alkoxyalkyl, Alkylthio, Alkylamino, Dialkylamino, Alkylcarbonyl etc.) bedeutet eine geradkettige oder verzweigte Alkylgruppe mit n bis m Kohlenstoffatomen, z.B. 1 bis 6 und insbesondere 1 bis 4 Kohlenstoffatomen. Beispiele für eine Alkylgruppe sind Methyl, Ethyl, n-Propyl, iso-Propyl, n-Butyl, 2-Butyl, iso-Butyl, tert-Butyl, n-Pentyl, 2-Pentyl, Neopentyl, n-Hexyl und dergleichen.

Der Ausdruck "gegebenenfalls substituiertes Cₙ-Cₘ-Alkyl" steht für einen Alkylrest mit n bis m C-Atomen, der teilweise oder vollständig durch Halogen, insbesondere durch Chlor oder Fluor substituiert sein kann und der einen oder mehrere, z. B. 1, 2 oder 3 von Halogen verschiedene Substituenten tragen kann, die ausgewählt sind unter CN, C₃-C₇-Cycloalkyl, C₃-C₇-Heterocycloalkyl, gegebenenfalls substituiertem Phenyl, OR¹¹, COOR¹¹, NR¹²R¹³, SO₂NR¹²R¹³, CONR¹²R¹³, O-CONR¹²R¹³, S-R¹⁴,SOR¹⁵, SO₂R¹⁵, OCOR¹⁶ und COR¹⁶. Hierin hat R¹¹ die für R¹ angegebene Bedeutung, R¹² die für R² angegebene Bedeutung, R¹³ die für R³ angegebene Bedeutung, R¹⁴ die für R⁴ angegebene Bedeutung, R¹⁵ die für R⁵ angegebene Bedeutung und R¹⁸ die für R⁶ angegebene Bedeutung. Insbesondere stehen R¹¹ - R¹⁸ für Wasserstoff. C₁-C₄-Alkyl, C₁-C₄-Halogenalkyl, C₃-C₇-Cycloalkyl, gegebenenfalls substituiertes Benzyl oder gegebenenfalls substituiertes Phenyl. Bevorzugte Substituenten an Alkyl sind unter OH, C₁-C₄-Alkoxy, Halogen, C₃-C₇-Cycloalkyl oder gegebenenfalls substituiertem Phenyl ausgewählt. Im Falle von OH, C₁-C₄-Alkoxy, C₃-C₇-Cycloalkyl und Phenyl ist es insbesondere nur ein Substituent. Derartige Reste werden im Folgenden auch als C,-C₄-Alkoxy-C₁-C₆-alkyl wie Methoxymethyl, 1- oder 2-Methoxyethyl, 1-Methoxy-1-methylethyl oder 2-Methoxy-1-methylethyl, 1-, 2- oder 3-Methoxypropyl, Ethoxymethyl, 1- oder 2-Ethoxyethyl, Hydroxy-C₁-C₆-alkyl, 1-Hydroxymethyl, 1- oder 2-Hydroxyethyl, 1-Hydroxy-1-methyletyl, 1-, 2- oder 3-Hydroxypropyl etc., C₃-C₆-Cycloalkyl C₁-C₈-alkyl wie Cyclopropylmethyl, Cyclohexylmethyl, oder Phenyl-C₁-C₆-alkyl bezeichnet. Im Falle von Halogen-Substituenten werden diese Reste auch als Halogenalkyl bezeichnet.

C₁-C₆-Halogenalkyl (auch in Resten wie C₁-C₆-Halogenalkoxy) steht für eine Alkylgruppe mit 1 bis 6 und insbesondere 1 bis 4 C-Atomen, wie oben definiert, in der alle oder ein Teil, z.B. 1, 2, 3, 4 oder 5 der Wasserstoffatome durch Halogenatome, insbesondere durch Chlor oder Fluor ersetzt sind. Bevorzugtes Halogenalkyl ist C₁-C₂-Fluoralkyl oder C₁-C₂-Fluorchloralkyl insbesondere CF₃, CHF₂, CF₂Cl, CH₂F, CH₂CF₃.

C₃-C₁₀-Cycloalkyl, auch in Resten wie Cycloalkylalkyl, Cycloalkylcarbonyl und Cycloalkylcarbonylalkyl steht für einen cycloaliphatischen Rest mit 3 bis 10 und vorzugsweise 3 bis 6 C-Atomen wie Cyclopropyl, Cyclobutyl, Cyclopentyl, Cyclohexyl, Cycloheptyl und Cyclooctyl.

C₃-C₁₀-Heterocycloalkyl, auch in Resten wie Heterocycloalkylalkyl, Heterocycloalkylcarbonyl und Heterocycloalkylcarbonylalkyl steht für einen gesättigten heterocyclischen Rest mit Ringgliedern, wobei 1, 2 oder 3 Ringglieder ein Heteroatom ausgewählt unter N, O und S sind wie Oxiranyl, Oxetanyl, Aziranyl, Azetanyl, Tetrahydrofurfuryl, Tetrahydrothienyl, Pyrrolidinyl, Pyrazolinyl, Imidazolinyl, Piperidinyl, Piperazinyl oder Morpholinyl.

C₄-C₁₀-Bicycloalkyl steht für einen bicycloaliphatischen Rest mit 4 bis 10 C-Atomen wie in Bicyclo[2.1.0]pentyl, Bicyclo[2.1.1 ]hexyl, Bicyclo[3.1.0]hexyl, Bicyclo[2.2.1]heptanyl, Bicyclo[3.2.0]heptanyl, Bicyclo[2.2.2]octanyl, Bicyclo[3.2.1]octanyl, Bicyclo[3.3.1]nonyl und Bicyclo[4.4.0]decyl.

C₆-C₁₀-Tricycloalkyl steht für einen tricycloaliphatischen Rest mit 6 bis 10 C-Atomen wie in Adamantyl.

C₂-C₆-Alkenyl steht für einen einfach ungesättigten linearen oder verzweigten Kohlenwasserstoffrest mit 2, 3, 4, 5 oder 6 C-Atomen, z.B. für Vinyl, Allyl (2-Propen-1-yl), 1-Propen-1-yl, 2-Propen-2-yl, Methallyl (2-Methylprop-2-en-1-yl) und dergleichen. C₃-C₄-Alkenyl steht insbesondere für Allyl, 1-Methylprop-2-en-1-yl, 2-Buten-1-yl, 3-Buten-1-yl, Methallyl, 2-Penten-1-yl, 3-Penten-1-yl, 4-Penten-1-yl, 1-Methylbut-2-en-1-yl, 2-Ethylprop-2-en-1-yl.

C₂-C₆-Halogenalkenyl steht für eine Alkenylgruppe wie oben definiert, in der alle oder ein Teil, z.B. 1, 2, 3, 4 oder 5 der Wasserstoffatome durch Halogenatome, insbesondere durch Chlor oder Fluor ersetzt sind.

C₂-C₆-Alkinyl steht für einen Kohlenwasserstoffrest mit 2, 3, 4, 5 oder 6 C-Atomen, der eine Dreifachbindung aufweist, z.B. für Propargyl (2-Propin-1-yl), 1-Methylprop-2-in-1-yl, 2-Butin-1-yl, 3-Butin-1-yl, 2-Pentin-1-yl, 1-Pentin-3-yl etc.

C₂-C₆-Halogenalkinyl steht für eine Alkenylgruppe wie oben definiert, in der alle oder ein Teil, z.B. 1, 2, 3, 4 oder 5 der Wasserstoffatome durch Halogenatome, insbesondere durch Chlor oder Fluor ersetzt sind.

Phenyl-C₁-C₄-alkyl steht für eine C₁-C₄-Alkylrest, wie oben definiert, in dem ein Wasserstoffatom durch einen Phenylrest ersetzt ist wie in Benzyl oder 2-Phenylethyl.

Gegebenenfalls substituiertes Phenyl steht für Phenyl, dass gegebenenfalls einen oder mehrere, z.B. 1, 2 oder 3 der nachfolgenden Substituenten aufweist: Halogen, Nitro, Cyano, gegebenenfalls substituiertes C₁-C₄-Alkyl, C₂-C₆-Alkenyl, C₂-C₆-Alkinyl, C₃-C₆-Cycloalkyl, C₁-C₄-Halogenalkyl, C₁-C₄-Alkoxy, OR²¹, COOR²¹, NR²²R²³, SO₂NR²²R²³, CONR²²R²³, O-CONR²²R²³, S-R²⁴, SOR²⁵, SO₂R²⁵, OCOR²⁸ und COR²⁶. Beispiele für geeignete Substituenten an Phenyl sind insbesondere Halogen, C₁-C₄-Alkyl, C₂-C₆-Alkenyl, C₂-C₆-Alkinyl, C₃-C₆-Cycloalkyl, C₁-C₄-Halogenalkyl, C₁-C₄-Alkoxy, C₁-C₄-Alkoxy-C₁-C₄-alkyl, Hydroxy-C₁-C₄-alkyl, Hydroxy, Nitro, NH₂, Cyano, COOH, C₁-C₄-Alkoxycarbonyl, C₁-C₄-Alkylcarbonyl, C₁-C₄-Alkylamino, Di-(C₁-C₄-alkyl)amino, C₁-C₄-Alkylsulfonyl, C₁-C₄-Alkylsulfonylamino und/oder C₁-C₄-Alkylaminosulfonyl. Hierin hat R²¹ die für R¹ angegebene Bedeutung, R²² die für R² angegebene Bedeutung, R²³ die für R³ angegebene Bedeutung, R²⁴ die für R⁴ angegebene Bedeutung, R²⁵ die für R⁵ angegebene Bedeutung und R²⁶ die für R⁶ angegebene Bedeutung. Insbesondere stehen R²¹ - R²⁶ für Wasserstoff. C₁-C₄-Alkyl, C₁-C₄-Halogenalkyl, C₃-C₇-Cycloalkyl, gegebenenfalls substituiertes Benzyl oder gegebenenfalls substituiertes Phenyl.

Der Begriff "Alkylen" umfasst grundsätzlich geradkettige oder verzweigte Reste mit vorzugsweise mit 2 bis 10 und besonders bevorzugt 3 bis 8 Kohlenstoffatomen wie Prop-1,2-ylen, Prop-1,3-ylen, But-1,2-ylen, But-1,3-ylen, But-1,4-ylen, 2-Methylprop-1,3-ylen, Pent-1,2-ylen, Pent-1,3-ylen, Pent-1,4-ylen, Pent-1,5-ylen, Pent-2,3-ylen, Pent-2,4-ylen, 1-Methylbut-1,4-ylen, 2-Methylbut-1,4-ylen, Hex-1,3-ylen, Hex-2,4-ylen, Hex-1,4-ylen, Hex-1,5-ylen, Hex-1,6-ylen und dergleichen. C₀-Alkylen steht für eine Einfachbindung, C₁-Alkylen für Methylen und C₂-Alkylen für 1,1-Ethylen oder 1,2-Ethylen.

Der Begriff 3 bis 8 gliedriges Heterocyclyl umfasst gesättigte (= Heterocycloalkyl), teilweise ungesättigte heterocyclische Reste und aromatische Heterocyclen (Heteroaryl) der Ringgröße 3, 4, 5, 6, 7 und 8, insbesondere der Ringgröße 5 oder 6, die 1, 2 oder 3 Heteroatome als Ringglieder aufweisen. Die Heteroatome sind dabei ausgewählt unter O, S und N.

Beispiele für gesättigtes 3- bis 8-gliedriges Heterocyclyl sind Oxiranyl, Oxetanyl, Aziranyl, Piperidinyl, Piperazinyl, Morpholinyl, Pyrrolidinyl, Oxazolidinyl, Tetrahydrofuryl, Dioxolanyl, Dioxanyl, Hexahydroazepinyl, Hexyhydrooxepinyl, und Hexahydrothiepinyl.

Beispiele für teilweise ungesättigtes 3- bis 8 gliedriges Heterocyclyl sind Di- und Tetrahydropyridinyl, Pyrrolinyl, Oxazolinyl, Dihydrofuryl, Tetrahydroazepinyl, Tetrahydrooxepinyl, und Tetrahydrothiepinyl.

Beispiele für 5- gliedrige heteroaromatische Reste (= 5-gliedriges Heteroaryl) sind solche mit 1, 2, 3 oder 4 Heteroatomen als Ringglieder, die unabhängig voneinander ausgewählt sind unter O, N und S, z.B. Pyrrol, Thiophen, Furan, Oxazol, Isoxazol, gewählt sind unter O, N und S, z.B. Pyrrol, Thiophen, Furan, Oxazol, Isoxazol, Thiazol, Isothiazol, Imidazol, 1,2,3-Thiadiazol, 1,2,4-Thiadiazol, 1,3,4-Thiadiazol, 1,2,3-Triazol, 1,2,4-Triazol, 1,3,4-Triazol, Tetrazol. Beispiele für 6-gliedrige heteroaromatische Reste (= 6-gliedriges Heteroaryl), die 1 oder 2 Stickstoffatome als Ringglieder aufweisen sind vor allem 2-, 3- oder 4-Pyridinyl, 2-, 4- oder 5-Pyrimidinyl, 2- oder 3-Pyrazinyl sowie 3-oder 4-Pyridazinyl. Die 6-gliedrigen heteroaromatischen Reste können die oben angegebenen Substituenten aufweisen und/oder mit einem nichtaromatischen oder aromatischen Carbocyclus, insbesondere einem Benzol- oder Cyclohexen-Ring condensiert sein wie in Benzo[b]pyridin (= Chinolin), Benzo[c]pyridin (Isochinolin), Benzo[b]pyrimidin (Chinazolin), Cinnolin, Phthalazin oder Chinoxalin. In den 5- oder 6-gliedrigen heteroaromatischen Resten Ar erfolgt die Bindung an die Gruppe (CH₂)ₚ vorzugsweise über ein Kohlenstoffatom.

Wenn die Alkylengruppe in D eine Carbonylgruppe umfasst, ist die Carbonylgruppe an das Stickstoffatom der Gruppe A oder an das Stickstoffatom der Gruppe NZ gebunden.

Besonders bevorzugt steht k für 4, 5 oder 6 und I für 3, 4 oder 5.

Sofern A für eine Gruppe 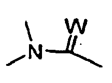 steht, dann bedeutet D vorzugsweise (CH₂)ₖ worin k die zuvor genannten Bedeutungen, insbesondere die als bevorzugt genannten Bedeutungen aufweist. W steht für Sauerstoff.

In einer ersten Ausführungsform der Erfindung steht B in den Formeln I und I' für eine Gruppe CR^{m}Rⁿ, worin R^{m} und Rⁿ die zuvor genannten Bedeutungen aufweisen und insbesondere für Wasserstoff oder C₁-C₄-Alkyl stehen. Insbesondere steht wenigstens einer der Reste R^{m} oder Rⁿ und speziell beide Reste R^{m} und Rⁿ für Wasserstoff.

In einer zweiten Ausführungsform der Erfindung steht B in den Formeln I und I' für eine Bindung.

Im Hinblick auf die Verwendung der erfindungsgemäßen Verbindungen I und I' als Dopamin-D₃-Rezeptor-Liganden sind solche Verbindungen I und I' bevorzugt, worin das Stickstoffatom der Gruppe A mit dem Kohlenstoffatom verbunden ist, das die Gruppe R^{Y} trägt.

Die Reste R^{v} und R^{w} stehen unabhängig voneinander insbesondere für Wasserstoff oder C₁-C₄-Alkyl. Insbesondere steht wenigstens einer der Reste R^{v} oder R^{w} und speziell beide Reste R^{v} und R^{w} für Wasserstoff.

Unter den Verbindungen der Formel I sind solche Verbindungen bevorzugt, worin R^{x} und R^{y} gemeinsam mit den C-Atomen, an die sie gebunden sind, für einen kondensierten Benzolring oder einen kondensierten 5- oder 6-gliedrigen, aromatischen Heterocyclus stehen, der 1, 2, 3 oder 4 Heteroatome aufweist, die ausgewählt sind unter N, O und S, wobei der kondensierte Phenylring sowie der kondensierte aromatische Heterocyclus 1, 2 oder 3 Substituenten aufweisen können, die ausgewählt sind unter C₁-C₄-Alkyl, C₁-C₄-Hydroxyalkyl, C₁-C₄-Alkoxy-C₁-C₄-alkyl, CN, OR¹, NR²R³, NO₂, SR⁴, SO₂R⁴, SO₂NR²R³, CONR²R³, COOR⁵, COR⁶, C₁-C₂-Fluoralkyl, C₁-C₂-Fluoralkoxy, C₂-C₆-Alkenyl, C₂-C₆-Alkinyl, C₂-C₆-Alkenyloxy, C₂-C₆-Atkinyloxy, C₃-C₆-Cycloalkyl und Halogen; worin R¹, R², R³, R⁴, R⁵ und R⁶ unabhängig voneinander die zuvor angegebenen Bedeutungen aufweisen. Bevorzugte Substituenten sind C₁-C₄-Alkyl, C₁-C₄-Halogenalkyl, C₁-C₄-Alkoxy und Halogen. Hierunter sind insbesondere solche Verbindungen der Formel I bevorzugt, worin R^{x} und R^{y} gemeinsam mit den C-Atomen, an die sie gebunden sind, für einen gegebenenfalls in der zuvor beschriebenen Weise substituierten, kondensierten Benzolring stehen.

Bei den Gruppen der Formel 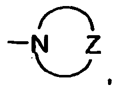 im Folgenden auch als NZ bezeichnet, handelt es sich um einen gesättigten oder einfach ungesättigten mono cyclischen Stickstoffheterocyclus, der gegebenenfalls ein weiteres Heteroatom als Ringglied umfasst, das unter Stickstoff, ausgewählt ist. Die Gruppe NZ kann eine Gruppe R^{a} aufweisen, Zusätzlich kann NZ eine C₁-C₄-Alkylgruppe, insbesondere Methylgruppe tragen.

Beispiele für geeignete Gruppen NZ sind die im folgenden angegebenen monocyclischen Reste.

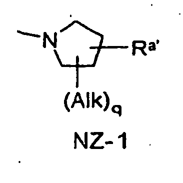

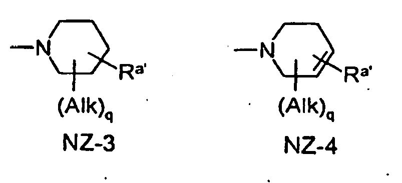

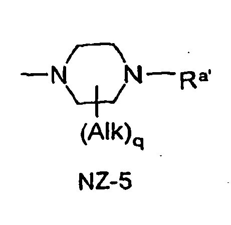

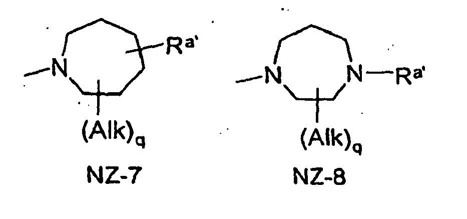

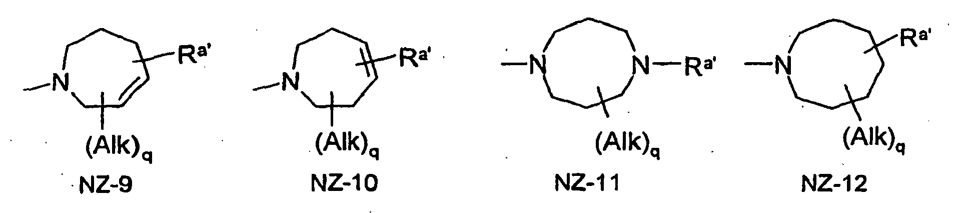

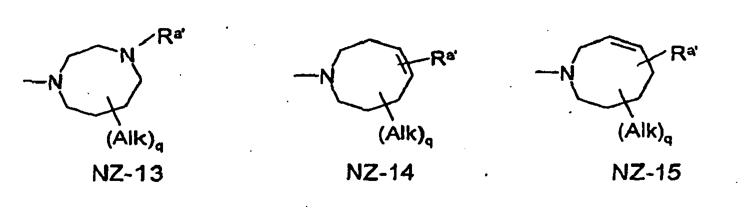

In den Resten NZ-1 bis NZ-15 weist R^{a'} die zuvor für R^{a} angegebenen Bedeutungen auf. Die Variable q steht für 0 oder 1 und Alk steht für eine Alkylgruppe mit 1 bis 4 C-Atomen. R^{a'} ist in den Resten NZ-3 bis NZ-5 und NZ-7 bis NZ-14 in der 4- oder in der 5-Postion, bezogen auf das Stickstoffatom, welches an D bindet, angeordnet.

Im Hinblick auf die Verwendung dererfindungsgemäßen Verbindungen als Dopamin-D₃-Rezeptor-Liganden sind solche Verbindungen 1 besonders bevorzugt, worin die Gruppe NZ als Rest R^{a} einen Rest E-Ar und insbesondere einen Rest (CH₂)ₚ-Ar aufweist. Hierin haben Ar und E die zuvor genannten Bedeutungen, und p steht für 0, 1, 2, 3 oder 4 und insbesondere für 0 oder 1.

Unter den erfindungsgemäßen Verbindungen I und I', in denen die Gruppe NZ als Rest R^{a} eine Gruppe der Formel (CH₂)ₚ-Ar aufweist, sind insbesondere solche Verbindungen bevorzugt worin p = 0 ist und Ar für Phenyl, Pyridyl, Pyrimidinyl oder s-Triazinyl steht, die 1, 2 oder 3 der vorgenannten Reste R^{b} aufweisen. Insbesondere steht Ar dann für einen Rest der Formel Ar-1 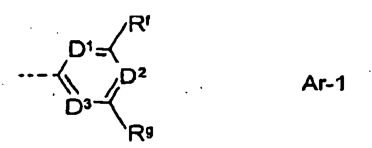 steht, worin die Variablen D¹ bis D³ unabhängig voneinander für N, CH oder C-R^{b} stehen. Hierin hat R^{b} eine der zuvor genannten Bedeutungen. R^{f} und R⁹ stehen unabhängig voneinander für Wasserstoff oder weisen eine der für R^{b} angegebenen Bedeutungen auf.

In einer ersten bevorzugten Ausführungsform der Erfindung steht in Formel Ar-1 wenigstens eine der Variablen D¹ bis D³ für N und die verbleibenden Variablen stehen für CH, wobei eine der Variablen D¹ bis D³ auch für C-R^{b} stehen kann, wenn eine der Variablen R¹ für Wasserstoff steht. Hierunter bevorzugt sind Verbindungen I und I', worin D' und speziell D¹ und D² für Stickstoff und die übrigen Variablen jeweils für CH stehen. Vorzugsweise stehen R¹ und R⁹ in dieser Ausführungsform unabhängig voneinander für die folgenden Gruppen: Wasserstoff, OR¹, NR²R³, CN, C₁-C₆-Alkyl, das gegebenenfalls durch OH, C₁-C₄-Alkoxy, Halogen oder Phenyl substituiert ist, C₂-C₆-Alkenyl, C₂-C₆-Alkinyl, C₃-C₆-Cycloalkyl, C₄-C₁₀-Bicycloalkyl, C₆-C₁₀-Tricycloalkyl, wobei die drei letztgenannten Gruppen gegebenenfalls durch Halogen oder C₁-C₄-Alkyl substituiert sein können, Halogen, CN, OR¹, 5- oder 6-gliedriges Heterocyclyl mit 1, 2 oder 3 Heteroatomen, ausgewählt unter O, S und N, und Phenyl, wobei Phenyl und Heterocyclyl gegebenenfalls ein oder zwei Substituenten tragen, die unabhängig voneinander ausgewählt sind unter C₁-C₄-Alkyl, C₁-C₄-Alkoxy, NR²R³, CN, C₁-C₂-Fluoralkyl und Halogen. Vorzugsweise ist R^{f} von Wasserstoff verschieden. Insbesondere sind sowohl R^{f} als auch R⁹ von Wasserstoff verschieden. Insbesondere steht R^{f} für C₁-C₆-Alkyl, besonders bevorzugt für verzweigtes C₃-C₆-Alkyl und speziell für tert.-Butyl. R^{g} ist vorzugsweise ausgewählt unter C₁-C₄-Alkyl, C₃-C₆-Cycloalkyl und C₁-C₂-Fluoralkyl. Besonders bevorzugt weisen R^{f} und R⁹ gemeinsam die als bevorzugt angegebenen Bedeutungen auf.

In einer anderen Ausführungsform der Erfindung stehen alle Variablen D¹ bis D³ in Ar-1 für CH oder eine Gruppe C-R^{b}. Vorzugsweise sind R^{f}, R⁹ und R^{b} in dieser Ausführungsform ausgewählt unter Wasserstoff, OR¹, NR²R³, CN, C₁-C₆-Alkyl, das gegebenenfalls durch OH, C₁-C₄-Alkoxy, Halogen oder Phenyl substituiert ist, C₂-C₆-Alkenyl, C₂-C₆-Alkinyl, C₃-C₆-Cycloalkyl, C₄-C₁₀-Bicycloalkyl, C₆-C₁₀-Tricycloalkyl, wobei die drei letztgenannten Gruppen gegebenenfalls durch Halogen oder C₁-C₄-Alkyl substituiert sein können, Halogen, CN, OR¹, 5- oder 6-gliedriges Heterocyclyl mit 1, 2 oder 3 Hetero-atomen, ausgewählt unter O, S und N, und Phenyl, wobei Phenyl und Heterocyclyl gegebenenfalls ein oder zwei Substituenten tragen, die unabhängig voneinander ausgewählt sind unter C₁-C₄-Alkyl, C₁-C₄-Alkoxy, NR²R³, CN, C₁-C₂-Fluoralkyl und Halogen. Besonders bevorzugt weist Ar-1 dann wenigstens einen von Wasserstoff verschiedenen Substituenten auf. Bevorzugte, von Wasserstoff verschiedene Substituenten sind in diesem Fall ausgewählt unter Halogen, speziell Chlor oder Fluor, C₁-C₄-Alkyl, C₁-C₄-Alkoxy, C₁-C₄-Alkoxy-C₁-C₄-alkyl, C₁-C₄-Halogenalkyl, C₁-C₄-Halogenalkoxy und CN. In einer besonders bevorzugten Ausführungsform steht dann Ar-1 für 2,3-Dichlorphenyl.

Unter den erfindungsgemäßen Verbindungen I und I', in denen die Gruppe NZ einen Rest R^{a} der Formel (CH₂)ₚ-Ar aufweist, sind weiterhin solche Verbindungen bevorzugt worin p = 1 ist und Ar die zuvor genannten Bedeutungen aufweist. Insbesondere steht Ar für Phenyl, Naphthyl, Pyridyl, Pyridinyl, Pyrazinyl, Pyridazinyl, Thienyl, Furyl, Pyrrolyl, Pyrazolyl, Imidazolyl, Oxazolyl, Isoxazolyl, Thiazolyl, Isothiazolyl, 1-Oxa-3,4-diazolyl oder 1-Thia-3,4-diazolyl, die unsubstituiert sind oder 1, 2 oder 3 der obengenannten Reste R^{b} aufweisen können. Speziell steht Ar dann für Phenyl, Pyridyl, Thiadiazolyl, Thienyl oder Imidazolyl, das 1, 2 oder 3 der obengenannten Reste R^{b} aufweisen kann. Bevorzugte Reste R^{b} sind dann insbesondere Halogen, speziell Chlor oder Fluor, Nitro, Cyano, C₁-C₄-Alkyl, C₁-C₄-Alkoxy, C₁-C₄-Halogenalkyl und/oder C₁-C₄-Halogenalkoxy.

In den Verbindungen I, in denen NZ einen Rest der Formel E-Ar trägt kann Ar, sofern es für Phenyl oder einen heteroaromatischen Rest steht, auch mit einem aromatischen oder heteroaromatischen 5- oder 6-gliedrigen Cyclus der oben genannten Art kondensiert sein, z.B. mit einem 5- oder 6-gliedrigen aromatischen oder nichtaromatischen Heterocyclus, der 1, 2 oder 3 unter O, N und S ausgewählte Heteroatome aufweist, z.B. mit Pyridin. Pyrimidin. Pyrazin, Pyridazin. Furan, Thiophen, Oxazol, Isoxazol, Thiazol, Isothiazol, 1,4-Dioxan, 1,4-Oxazinan oder 1,3-Dioxolan, wie in Chinolin, isochinolin, 4H-Chinolizin, 1,5-, 1,6-,1,7-,1,8-,2,6-oder2,7-Naphthyridin, Indol, Indolizin, 1-oder 2-Benzofuran, 1- oder 2-Benzothiophen, 1,3-Benzoxazol, Benz[1,2-b und 1,2-c]oxazol, 1,3-Benzothiazol, 1,3-Benzirnidazol, Benzo[1,2-b und 1,2-c]isothiazol, Chinazolin, Chromen, Chroman, 1,4-Benzopiperazin, 1- oder 2-Benzopiperidin, Benzo[1,4-b]oxazinan, Benzo[1,3-b]dioxotan oder Benzo[1,4-b]dioxan. Phenyl und die aromatischen Heterocyclen, insbesondere Phenyl und Pyridyl können auch mit einem 5- oder 6-gliedrigen, Carbocylus kondensiert sein, z.B. mit Benzol, Cyclohex(adi)en Cylopent(adi)en, wie in Naphthanlin, Indan, Inden, Chinolin, isochinolin, Di- oder Tetrahydronaphthalin. In derartigen Resten erfolgt die Bindung von Ar über den Phenyl-, Pyridyl- oder Pyrimidinylteil des bicyclischen Rests.

Die Reste NZ gehorchen der Formel: 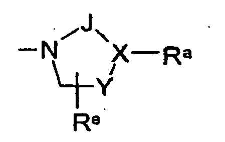 worin
- R^{a}: die zuvor genannten Bedeutungen und insbesondere die als bevorzugt angegebenen Bedeutungen aufweist;
- J: für CH₂, CH₂-CH₂ oder CH₂-CH₂-CH₂ und insbesondere für CH₂-CH₂ steht;
- X: für CH oder N und
- Y: für CH₂, CH₂-CH₂ oder CH₂-CH₂-CH₂ stehen oder Y-X gemeinsam CH=C oder CH₂-CH=C bedeuten, wobei solche Reste bevorzugt sind, worin X für N steht;
- R^{e}: Wasserstoff oder C₁-C₄-Alkyl bedeutet und insbesondere für Wasserstoff steht.

Beispiele für derartige Reste sind die zuvor genannten Reste NZ, worunter die Reste NZ-3, NZ-4 und NZ 5 besonders bevorzugt sind. Ganz besonders bevorzugt steht NZ für den Rest NZ-5.

In einer ersten bevorzugten Ausführungsform der Erfindung steht NZ für einen Rest der Formel NZ-A 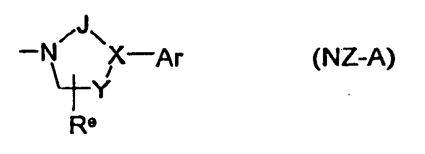 worin J, X, Y, R^{e} und Ar die zuvor genannten und insbesondere die als bevorzugt genannten Bedeutungen haben und Ar insbesondere für Ar-1 steht.

In einer zweiten bevorzugten Ausführungsform der Erfindung steht NZ für einen Rest der Formel NZ-B 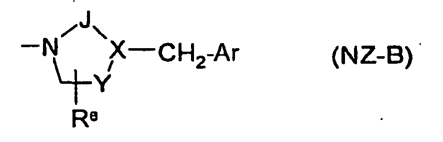 worin J, X, Y, R^{e} und Ar die zuvor genannten und insbesondere die als bevorzugt genannten Bedeutungen haben. Insbesondere steht Ar für Phenyl, Naphthyl, Pyridyl, Pyridinyl, Pyrazinyl, Pyridazinyl, Thienyl, Furyl, Pyrrolyl, Pyrazolyl, Imidazolyl, Oxazolyl, Isoxazolyl, Thiazolyl, Isothiazolyl, 1-Oxa-3,4-diazolyl oder 1-Thia-3;4-diazolyl, die unsubstituiert sind oder 1, 2 oder 3 der obengenannten Reste R^{b} aufweisen können. Speziell steht Ar dann für Phenyl, Pyridyl, Thienyl oder Imidazolyl, das 1, 2 oder 3 der obengenannten Reste R^{b} aufweisen kann. Bevorzugte Reste R^{b} sind dann insbesondere Halogen, speziell Chlor oder Fluor, Nitro, Cyano, C₁-C₄-Alkyl, C₁-C₄-Alkoxy, C₁-C₄-Halogenalkyl und/oder C₁-C₄-Halogenalkoxy.

In den Verbindungen der Formel I steht die Gruppe der Formel 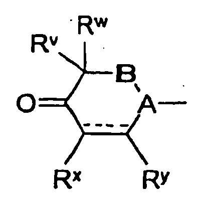 vorzugsweise für die im folgenden angegebene Gruppe A

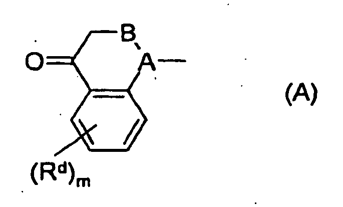

In Formel A haben die Variablen A und B die zuvor genannten, insbesondere die als bevorzugt genannten Bedeutungen. Die Variable A steht in Formel A für N-C(O), worin das C-Atom an die Variable B gebunden ist. B in Formel A steht insbesondere für CH₂, Die Variable m steht für 0, 1, 2 oder 3, insbesondere 0 oder 1. R^{d} steht unabhängig voneinander für C₁-C₄-Alkyl, C₁-C₄-Hydroxyalkyl, C₁-C₄ Alkoxy-C₁-C₄-alkyl, CN, OR¹, NR²R³, NO₂, SR⁴, SO₂R⁴, SO₂NR²R³, CONR²R³, COOR⁵, COR⁶, C₁-C₂-Fluoralkyl, C₁-C₂-Fluoralkoxy, C₂-C₆-Alkenyl, C₂-C₆-Alkinyl, C₂-C₆-Alkenyloxy, C₂-C₆-Alkinyloxy, C₃-C₆-Cycloalkyl oder Halogen und ist insbesondere ausgewählt unter C₁-C₄-Alkyl, C₁-C₄-Alkoxy, C₁-C₂-Fluoralkyl und Halogen. Verbindungen der allgemeinen Formel I bzw. ihre Tautomere I', die eine Gruppe A aufweisen, werden im Folgenden auch als Verbindungen I-A bzw. I-A' bezeichnet.

Unter den Gruppen der Formeln A sind insbesondere die Gruppen A1 und A2 zu nennen:

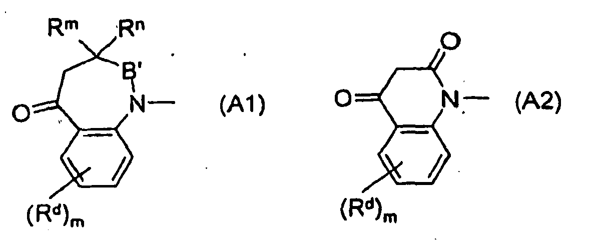

In den Formeln A1, und A2 weisen die Variablen m und R^{d} die zuvor genannten, insbesondere die als bevorzugt genannten Bedeutungen auf. In Formel A1 haben R^{m}, und Rⁿ die zuvor angegebenen Bedeutungen und insbesondere die als bevorzugt angegebenen Bedeutungen. Insbesondere steht wenigstens einer der Reste R^{m} oder Rⁿ und insbesondere beide Reste R^{m} und Rⁿ für Wasserstoff. B' steht für CO. Unter den Verbindungen IA bzw. den Tautomeren IA' sind insbesondere solche Verbindungen bevorzugt, welche als Gruppe A eine Gruppe der Formeln A1 oder A2 aufweisen.

Unter den Verbindungen der Formel IA betrifft eine bevorzugte Ausführungsform Verbindungen der nachstehend definierten Formel I-Aa und deren Tautomere I-Aa':

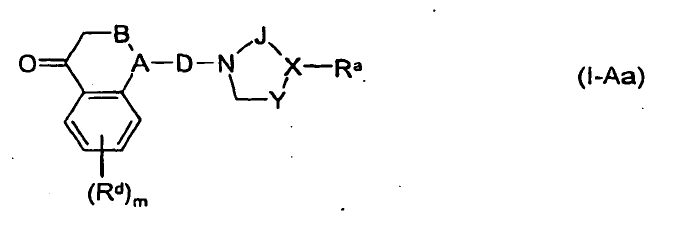

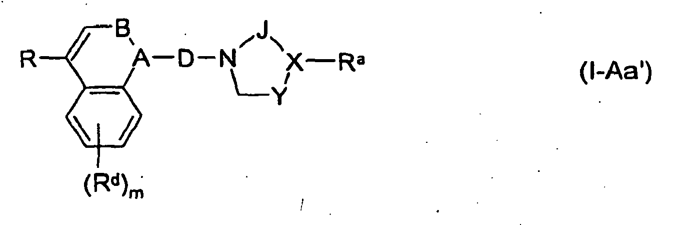

In den Formeln I-Aa und I-Aa' haben A, B, D, m, J, X, Y, R, R^{a} und R^{d} die zuvor angegebenen Bedeutungen und insbesondere die als bevorzugt angegebenen Bedeutungen.

Insbesondere steht J in den Formeln I-Aa und I-Aa' für CH₂-CH₂. Die Variable X steht in den Formeln I-Aa und I-Aa' insbesondere für N und Y steht insbesondere für CH₂.

Unter den Verbindungen I-Aa und I-Aa' sind solche besonders bevorzugt, worin D eine Gruppe (CH₂)ₖ oder eine Gruppe C(O)(CH₂)₁ bedeutet, worin und I die zuvor genannten Bedeutungen aufweisen, wobei k insbesondere für 4, 5 oder 6 und I insbesondere für 3, 4 oder 5 stehen.

Unter den Verbindungen I-Aa und 1-Aa' sind solche besonders bevorzugt, worin B für CH₂ steht.

Unter den Verbindungen I-Aa und I-Aa' sind solche besonders bevorzugt, worin R^{a} für eine Gruppe (CH₂)ₚ-Ar steht, worin p und Ar die zuvor angegebenen Bedeutungen aufweisen, wobei insbesondere p = 0 oder 1 gilt.

Für p = 0 weist Ar insbesondere die im Zusammenhang mit der Gruppe NZ-A angegebenen Bedeutungen auf. Für p = 1 hat Ar insbesondere die in Zusammenhang mit der Gruppe NZ-B angegebenen Bedeutungen.

In Substituenten OR¹, OR¹¹ und OR²¹ stehen R¹, R¹¹ und R²¹ häufig für H, C₁-C₄-Alkyl, CF₃, CHF₂ oder Phenyl. Insbesondere bevorzugt steht OR¹, OR¹¹ bwz. OR²¹ für Methoxy, Trifluormethoxy oder Phenoxy.

In Substituenten CONR²R³ (und analog in CONR¹²R¹³ und CONR²²R²³) steht R² vorzugsweise für H oder C₁-C₄-Alkyl und R³ vorzugsweise für H, C₁-C₄-Alkyl oder COR⁷. Insbesondere bevorzugt stehen CONR²R³, CONR¹²R¹³ bzw. CONR²²R²³ für CONH₂, CONHCH₃, CON(CH₃)₂ oder CONHCOCH₃.

In Substituenten NR²R³ (und analog in NR¹²R¹³ und NR²²R²³) steht R² vorzugsweise für H, C₁-C₄-Alkyl oder phenylsubstituiertes C₁-C₄-Alkyl und R³ für H, C₁-C₄-Alkyl oder COR⁷. Insbesondere bevorzugt stehen NR²R³, NR¹²R¹³ bzw. NR²²R²³ für NH₂, NHCH₃, N(CH₃)₂, NH-Benzyl oder NHCOCH₃.

In Substituenten SO₂NR²R³ (und analog in SO₂NR¹²R¹³ und SO₂NR²²R²³) steht R² vorzugsweise für H oder C₁-C₄-Alkyl und R³ vorzugsweise für H, C₁-C₄-Alkyl oder COR⁷. Insbesondere bevorzugt stehen SO₂NR²R³, SO₂NR¹²R¹³ bzw. SO₂NR²²R²³ für Sulfa-moyl.

Sofern R², R³ in den Substituenten NR²R³, CONR²R³, SO₂NR²R³ (analog in CONR¹²R¹³, CONR²²R²³, NR¹²R¹³, NR²²R²³, SO₂NR¹²R¹³ und SO₂NR²²R²³) gemeinsam mit dem Stickstoffatom, an das sie gebunden sind, für einen 5- oder 6-gliedrigen gesättigten oder ungesättigten N-Heterocyclus stehen, bedeuten die Gruppen NR²R³, NR¹²R¹³ und NR²²R²³ in diesen Resten z.B. N-Pyrrolidinyl, N-Piperidinyl, Morpholin-4-yl oder 4-Methylpiperazin-1-yl.

In Substituenten SR⁴, SR¹⁴ und SR²⁴ stehen R⁴, R¹⁴ bzw. R²⁴ vorzugsweise für C₁-C₄-Alkyl. Insbesondere bevorzugt steht SR⁴ für Thiomethyl.

In Substituenten SO₂R⁴, SO₂R¹⁴ und SO₂R²⁴stehen R⁴, R¹⁴ bzw. R²⁴ vorzugsweise für C₁-C₄-Alkyl, C₁-C₄-Halogenalkyl oder-Phenyl, das gegebenenfalls eine C₁-C₄-Alkylgruppe als Substituenten aufweist. Insbesondere bevorzugt stehen SO₂R⁴, SO₂R¹⁴ und SO₂R²⁴ für Methylsulfonyl.

In Substituenten COOR⁵, COOR¹⁵ und COOR²⁵ stehen R⁵, R¹⁵ bzw. R²⁵ häufig für H oder C₁-C₄-Alkyl. Insbesondere bevorzugt stehen CO0R⁵, COOR¹⁵ und COOR²⁵ für C₁-C₄-Alkoxycarbonyl, wie Methoxycarbonyl, Ethoxycarbonyl, n-Propoxycarbonyl, i-Propoxycarbonyl, n-Butoxycarbonyl oder t-Butoxycarbonyl.

In Substituenten COR⁶ (analog in COR¹⁶ und COR²⁸) steht R⁶ vorzugsweise für H, C₁-C₄-Alkyl oder ggf. substituiertes Phenyl. Insbesondere bevorzugt stehen COR⁸, COR¹⁶ und COR²⁶ für Formyl, Acetyl oder Benzoyl.

In Substituenten O-COR⁶ (analog in O-COR¹⁶ und O-COR²⁹) steht R⁶ vorzugsweise für H, C₁-C₄-Alkyl oder ggf. substituiertes Phenyl. Insbesondere bevorzugt stehen OCOR⁶, O-COR¹⁸ und O-COR²⁸ für Formyloxy, Acetyloxy oder Benzoyloxy.

In Substituenten COR⁷ (analog in COR¹⁷ und COR²⁷) steht R⁷ vorzugsweise für H, C₁-C₄-Alkyl oder ggf. substituiertes Phenyl. Insbesondere bevorzugt stehen COR⁷, COR¹⁷ und COR²⁷ für Formyl, Acetyl oder Benzoyl.

In der Gruppe NR⁸ steht R⁸ vorzugsweise für Wasserstoff oder Methyl.

In Substituenten COR⁹ steht R⁹ vorzugsweise für H, C₁-C₄-Alkyl oder ggf. substituiertes Phenyl. Insbesondere bevorzugt steht COR⁹ für Formyl, Acetyl oder Benzoyl.

Im Hinblick auf die Verwendung der erfindungsgemäßen Verbindungen als Dopamin-D₃-Rezeptor-Liganden sind die Verbindungen IA und insbesondere die Verbindungen IAa besonders bevorzugt.

Ganz besonders bevorzugt sind die Verbindungen der Formel I-Aa.a, worin die Variablen D, E und Ar die oben genannten, insbesondere die als bevorzugt genannten, Bedeutungen aufweisen. Beispiele für derartige Verbindungen sind die Verbindungen I-Aa.a.1 bis I-Aa.a.708, in denen die Variablen D, E und Ar gemeinsam die in einer Zeile der Tabelle A angegebenen Bedeutungen aufweisen.

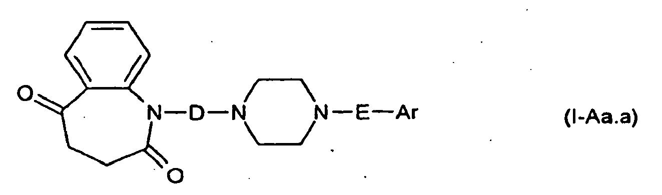

**Tabelle A:**

| | D | E | Ar |
|---|---|---|---|
| A-1 | CH₂-CH₂-CH₂-CH₂ | - | 2-tert.-Butyl-4-trifluormethylpyrimidin-6-yl |
| A-2 | CH₂-CH₂-CH₂-CH₂ | - | 2-tert.-Butyl-4-difluormethylpyrimidin-6-yl |
| A-3 | CH₂-CH₂-CH₂-CH₂ | - | 2-tert.-Butyl-4-phenylpyrimidin-6-yl |
| A-4 | CH₂-CH₂-CH₂CH₂ | - | 2-tert.-Butyl-4-methylpyrimidin-6-yl |
| A-5 | CH₂-CH₂-CH₂-CH₂ | - | 2-tert.-Butyl-4-ethylpyrimidin-6-yt |
| A-6 | CH₂-CH₂-CH₂-CH₂ | - | 2-tert.-Butyl-4-n-propylpyrimidin-6-yl |
| A-7 | CH₂-CH₂-CH₂-CH₂ | - | 2-tert.-Butyt-4-isopropylpyrimidin-6-yl |
| A-8 | CH₂-CH₂-CH₂-CH₂ | - | 2,4-Bis(tert.-butyl)pyrimidin-6-yl |
| A-9 | CH₂-CH₂-CH₂-CH₂ | - | 2-tert.-Butyl-4-cyclopropylpyrimidin-6-yl |
| A-10 | CH₂-CH₂-CH₂-CH₂ | - | 2-tert.-Butyl-4-cyclobutylpyrimidin-6-yl |
| A-51 | C(O)-CH₂-CH₂-CH₂ | - | 2-tert.-Butyl-4-trifluormethylpyrimidin-6-yl |
| A-52 | C(O)-CH₂-CH₂-CH₂ | - | 2-tert.-Butyl-4-difluormethylpyrimidin-6-yl |
| A-53 | C(O)-CH₂-CH₂-CH₂ | - | 2-tert.-Butyl-4-phenylpyrimidin-6-yl |
| A-54 | C(O)-CH₂-CH₂-CH₂ | - | 2-tert.-Butyl-4-methylpyrimidin-6-yl |
| A-55 | C(O)-CH₂-CH₂-CH₂ | - | 2-tert.-Butyl-4-ethylpyrimidin-6-yl |
| A-56 | C(O)-CH₂-CH₂-CH₂ | - | 2-tert.-Butyl-4-n-propylpyrimidin-6-yl |
| A-57 | C(O)-CH₂-CH₂-CH₂ | - | 2-tert.-Butyl-4-isopropylpyrimidin-6-yl |
| A-58 | C(O)-CH₂-CH₂-CH₂ | - | 2,4-Bis(tert.-butyl)pyrimidin-6-yl |
| A-59 | C(O)-CH₂-CH₂-CH₂ | - | 2-tert.-Butyl-4-cyclopropylpyrimidin-6-yl. |
| A-60 | C(O)-CH₂-CH₂-CH₂ | - | 2-tert.-Butyl-4-cyclobutylpyrimidin-6-yl |
| A-61 | C(O)-CH₂-CH₂-CH₂-CH₂ | - | 2-tert.-Butyl-4-trifluormethylpyrimidln-6-yl |
| A-62 | C(O)-CH₂-CH₂-CH₂-CH₂ | - | 2-tert.-Butyl-4-difluormethylpyrimidin-6-yl |
| A-63 | C(O)-CH₂-CH₂-CH₂-CH₂ | - | 2-tert.-Butyl-4-phenylpyrimidin-6-yl |
| A-64 | C(O)-CH₂-CH₂-CH₂-CH₂ | - | 2-tert.-Butyl-4-methylpyrimidin-6-yl |
| A-65 | C(O)-CH₂-CH₂-CH₂-CH₂ | - | 2-tert.-Butyl-4-ethylpyrimidin-6-yl |
| A-66 | C(O)-CH₂-CH₂-CH₂-CH₂ | - | 2-tert.-Butyl-4-n-propylpyrimidin-6-yl |
| A-67 | C(O)-CH₂-CH₂-CH₂-CH₂ | - | 2-tert.-Butyl-4-isopropylpyrimidin-6-yl |
| A-68 | C(O)-CH₂-CH₂-CH₂-CH₂ | - | 2,4-Bis(tert.-butyl)pyrimidin-6-yl |
| A-69 | C(O)-CH₂-CH₂-CH₂-CH₂ | - | 2-tert.-Butyl-4-cyclopropylpyrimidin-6-yl |
| A-70 | C(O)-CH₂-CH₂-CH₂-CH₂ | - | 2-tert.-Butyl-4-cyclobutylpyrimidin-6-yl |
| A-71 | CH₂-CH₂-CH₂-CH₂ | - | 2-tert.-Butyl-4-trifluormethylpyridin-6-yl |
| A-72 | CH₂-CH₂-CH₂-CH₂ | - | 2-tert.-Butyl-4-difluormethylpyridin-6-yl |
| A-73 | CH₂-CH₂-CH₂-CH₂ | - | 2-tert.-Butyl-4-phenylpyridin-6-yl |
| A-74 | CH₂-CH₂-CH₂-CH₂ | - | 2-tert.-Butyl-4-methylpyridin-6-yl |
| A-75 | CH₂-CH₂-CH₂-CH₂ | - | 2-tert.-Butyl-4-ethylpyridin-6-yl |
| A-76 | CH₂-CH₂-CH₂-CH₂ | - | 2-tert.-Butyl-4-n-propylpyridin-6-yl |
| A-77 | CH₂-CH₂-CH₂-CH₂ | - | 2-tert.-Butyl-4-isopropylpyridin-6-yl |
| A-78 | CH₂-CH₂-CH₂-CH₂ | - | 2,4-Bis(tert.-butyl)pyridin-6-yl |
| A-79 | CH₂-CH₂-CH₂-CH₂ | - | 2-tert.-Butyl-4-cyclopropylpyridin-6-yl |
| A-80 | CH₂-CH₂-CH₂-CH₂ | - | 2-tert.-Butyl-4-cyclobutylpyridin-6-yl |
| A-121 | C(O)-CH₂-CH₂-CH₂ | - | 2-tert.-Butyl-4-trifluormethylpyridin-6-yl |
| A-122 | C(O)-CH₂-CH₂-CH₂ | - | 2-tert.-Butyl-4-difluormethylpyridin-6-yl |
| A-123 | C(O)-CH₂-CH₂-CH₂ | - | 2-tert.-Butyl-4-phenylpyridin-6-yl |
| A-124 | C(O)-CH₂-CH₂-CH₂ | - | 2-tert.-Butyl-4-methylpyridin-6-yl |
| A-125 | C(O)-CH₂-CH₂-CH₂ | - | 2-tert.-Butyl-4-ethylpyridin-6-yl |
| A-126 | C(O)-CH₂-CH₂-CH₂ | - | 2-tert.-Butyl-4-n-propylpyridin-6-yl |
| A-127 | C(O)-CH₂-CH₂-CH₂ | - | 2-tert.-Butyl-4-isopropylpyridin-6-yl |
| A-128 | C(O)-CH₂-CH₂-CH₂ | - | 2,4-Bis(tert.-butyl)pyridin-6-yl |
| A-129 | C(O)-CH₂-CH₂-CH₂ | - | 2-tert.-Butyl-4-cyclopropylpyridin-6-yl |
| A-130 | C(O)-CH₂-CH₂-CH₂ | - | 2-tert.-Butyl-4-cyclobutylpyridin-6-yl |
| A-131 | C(O)-CH₂-CH₂-CH₂-CH₂ | - | 2-tert.-Butyl-4-trifluormethylpyridin-6-yl |
| A-132 | C(O)-CH₂-CH₂-CH₂-CH₂ | - | 2-tert.-Butyl-4-difluormethylpyridin-6-yl |
| A-133 | C(O)-CH₂-CH₂-CH₂-CH₂ | - | 2-tert.-Butyl-4-phenylpyridin-6-yl |
| A-134 | C(O)-CH₂-CH₂-CH₂-CH₂ | - | 2-tert.-Butyl-4-methylpyridin-6-yl |
| A-135 | C(O)-CH₂-CH₂-CH₂-CH₂ | - | 2-tert.-Butyl-4-ethylpyridin-6-yl |
| A-136 | C(O)-CH₂-CH₂-CH₂-CH₂ | - | 2-tert.-Butyl-4-n-propylpyridin-6-yl |
| A-137 | C(O)-CH₂-CH₂-CH₂-CH₂ | - | 2-tert.-Butyl-4-isopropylpyridin-6-yl |
| A-138 | C(O)-CH₂-CH₂-CH₂-CH₂ | - | 2,4-Bis(tert.-butyl)pyridin-6-yl |
| A-139 | C(O)-CH₂-CH₂-CH₂-CH₂ | - | 2-tert.-Butyl-4-cyclopropylpyridin-6-yl |
| A-140 | C(O)-CH₂-CH₂-CH₂-CH₂ | - | 2-tert.-Butyl-4-cyclobutylpyridin-6-yl |
| A-141 | CH₂-CH₂-CH₂-CH₂ | - | 2-tert.-Butyl-4-trifluormethyltriazin-6-yl |
| A-142 | CH₂-CH₂-CH₂-CH₂ | - | 2-tert-Butyl-4-difluormethyltriazin-6-yl |
| A-143 | CH₂-CH₂-CH₂-CH₂ | - | 2-tert-Butyl-4-phenyltriazin-6-yl |
| A-144 | CH₂-CH₂-CH₂-CH₂ | - | 2-tert-Butyl-4-methyltriazin-6-yl |
| A-145 | CH₂-CH₂-CH₂-CH₂ | - | 2-tert.-Butyl-4-ethyltriazin-6-yl |
| A-146 | CH₂-CH₂-CH₂-CH₂ | - | 2-tert.-Butyl-4-n-propyltriazin-6-yl |
| A-147 | CH₂-CH₂-CH₂-CH₂ | - | 2-tert.-Butyl-4-isopropyltriazin-6-yl |
| A-148 | CH₂-CH₂-CH₂-CH₂ | - | 2,4-Bis(tert.-butyl)-triazin-6-yl |
| A-149 | CH₂-CH₂-CH₂-CH₂ | - | 2-tert.-Butyl-4-cyclopropyltriazin-6-yl |
| A-150 | CH₂-CH₂-CH₂-CH₂ | - | 2-tert.-Butyl-4-cyclobutyltriazin-6-yl |
| A-191 | C(O)-CH₂-CH₂-CH₂ | - | 2-tert.-Butyl-4-trifluormethyltriazin-6-yl |
| A-192 | C(O)-CH₂-CH₂-CH₂ | - | 2-tert.-Butyl-4-difluormethyltriazin-6-yl |
| A-193 | C(O)-CH₂-CH₂-CH₂ | - | 2-tert.-Butyl-4-phenyltriazin-6-yl |
| A-194 | C(O)-CH₂-CH₂-CH₂ | - | 2-tert.-Butyl-4-methyltriazin-6-yl |
| A-195 | C(O)-CH₂-CH₂-CH₂ | - | 2-tert.-t3utyl-4-ethyltriazin-6-yl |
| A-196 | C(O)-CH₂-CH₂-CH₂ | - | 2-tert.-Butyl-4-n-propyltriazin-6-yl |
| A-197 | C(O)-CH₂-CH₂-CH₂ | - | 2-tert.-Butyl-4-isopropyltriazin-6-yl |
| A-198 | C(O)-CH₂-CH₂-CH₂ | - | 2,4-Bis(tert.-butyl)-triazin-6-yl |
| A-199 | C(O)-CH₂-CH₂-CH₂ | - | 2-tert.-Butyl-4-cyclopropyltriazin-6-yl |
| A-200 | C(O)-CH₂-CH₂-CH₂ | - | 2-tert.-Butyl-4-cyclobutyltriazin-6-yl |
| A-201 | C(O)-CH₂-CH₂-CH₂-CH₂ | - | 2-tert.-Butyl-4-trifluormethiyltriazin-6-yl |
| A-202 | C(O)-CH₂-CH₂-CH₂-CH₂ | - | 2-tert.-Butyl-4-difluormethyltriazin-6-yl |
| A-203 | C(O)-CH₂-CH₂-CH₂-CH₂ | - | 2-tert.-Butyl-4-phenyltriazin-6-yl |
| A-204 | C(O)-CH₂-CH₂-CH₂-CH₂ | - | 2-tert.-Butyl-4-methyltriazin-6-yl |
| A-205 | C(O)-CH₂-CH₂-CH₂-CH₂ | - | 2-tert.-Butyl-4-ethyltriazin-6-yl |
| A-206 | C(O)-CH₂-CH₂-CH₂-CH₂ | - | 2-tert.-Butyl-4-n-propyltriazin-6-yl |
| A-207 | C(O)-CH₂-CH₂-CH₂-CH₂ | - | 2-tert.-Butyl-4-isopropyltriazin-6-yl |
| A-208 | C(O)-CH₂-CH₂-CH₂-CH₂ | - | 2,4-Bis(tert.-butyl)-triazin-6-yl |
| A-209 | C(O)-CH₂-CH₂-CH₂-CH₂ | - | 2-tert.-Butyl-4-cyclopropyltriazin-6-yl |
| A-210 | C(O)-CH₂-CH₂-CH₂-CH₂ | - | 2-tert.-Butyl-4-cyclobutyltriazin-6-yl |
| A-211 | CH₂-CH₂-CH₂-CH₂ | - | 4-tert-Butyl-2-trifluormethylpyridin-6-yl |
| A-212 | CH₂-CH₂-CH₂-CH₂ | - | 4-tert.-Butyl-2-difluormethylpyridin-6-yl |
| A-213 | CH₂-CH₂-CH₂-CH₂ | - | 4-tert.-Butyl-2-phanylpyridin-6-yl |
| A-214 | CH₂-CH₂-CH₂-CH₂ | - | 4-tert.-Butyl-2-methylpyridin-6-yl |
| A-215 | CH₂-CH₂-CH₂-CH₂ | - | 4-tert.-Butyl-2-ethylpyridin-6-yl |
| A-216 | CH₂-CH₂-CH₂-CH₂ | - | 4-tert.-Butyl-2-n-propylpyridin-6-yl |
| A-217 | CH₂-CH₂-CH₂-CH₂ | - | 4-tert.-Butyl-2-isopropylpyridin-6-yl |
| A-218 | CH₂-CH₂-CH₂-CH₂ | - | 4-tert.-Butyl-2-cyclopropylpyridin-6-yl |
| A-219 | CH₂-CH₂-CH₂-CH₂ | - | 4-tert.-Butyl-2-cyclobutylpyridin-6-yl |
| A-256 | C(O)-CH₂-CH₂-CH₂ | - | 4-tert.-Butyl-2-trifluormethylpyridin-6-yl |
| A-257 | C(O)-CH₂-CH₂-CH₂ | - | 4-tert.-Butyl-2-difluormethylpyridin-6-yl |
| A-258 | C(O)-CH₂-CH₂-CH₂ | - | 4-tert.-Butyl-2-phenylpyridin-6-yl |
| A-259 | C(O)-CH₂-CH₂-CH₂ | - | 4-tert.-Butyl-2-methylpyridin-6-yl |
| A-260 | C(O)-CH₂-CH₂-CH₂ | - | 4-tert.-Butyl-2-ethylpyridin-6-yl |
| A-261 | C(O)-CH₂-CH₂-CH₂ | - | 4-tert.-Butyl-2-n-propylpyridin-6-yl |
| A-262 | C(O)-CH₂-CH₂-CH₂ | - | 4-tert.-Butyl-2-isopropylpyridin-6-yl |
| A-263 | C(O)-CH₂-CH₂-CH₂ | - | 4-tert.-Butyl-2-cyclopropylpyridin-6-yl |
| A-264 | C(O)-CH₂-CH₂-CH₂ | - | 4-tert.-Butyl-2-cyclobutylpyridin-6-yl |
| A-265 | C(O)-CH₂-CH₂-CH₂-CH₂ | - | 4-tert-Butyl-2-trifluormethylpyridiri-6-yl |
| A-266 | C(O)-CH₂-CH₂-CH₂-CH₂ | - | 4-tert.-Butyl-2-difluormethylpyridin-6-yl |
| A-267 | C(O)-CH₂-CH₂-CH₂-CH₂ | - | 4-tert.-Butyl-2-phenylpyridin-6-yl |
| A-268 | C(O)-CH₂-CH₂-CH₂-CH₂ | - | 4-tert.-Butyl-2-methylpyridin-6-yl |
| A-269 | C(O)-CH₂-CH₂-CH₂-CH₂ | - | 4-tert.-Butyl-2-ethylpyridin-6-yl |
| A-270 | C(O)-CH₂-CH₂-CH₂-CH₂ | - | 4-tert.-Butyl-2-n-propylpyridin-6-yl |
| A-271 | C(O)-CH₂-CH₂-CH₂-CH₂ | - | 4-tert.-Butyl-2-isopropylpyridin-6-yl |
| A-272 | C(O)-CH₂-CH₂-CH₂-CH₂ | - | 4-tert.-Butyl-2-cyclopropytpyridin-6-yl |
| A-273 | C(O)-CH₂-CH₂-CH₂-CH₂ | - | 4-tert.-Butyl-2-cyclobutylpyridin-6-yl |
| A-274 | CH₂-CH₂-CH₂-CH₂ | - | 2-tert.-Butyl-6-trifluormethylpyrimidin-4-yl |
| A-275 | CH₂-CH₂-CH₂-CH₂ | - | 2-tert.-Butyl-6-difluormethylpyrimidin-4-yl |
| A-276 | CH₂-CH₂-CH₂-CH₂ | - | 2-tert.-Butyl-6-phenylpyrimidin-4-yl |
| A-277 | CH₂-CH₂-CH₂-CH₂ | - | 2-tert.-Butyl-6-methylpyrimidin-4-yl |
| A-278 | CH₂-CH₂-CH₂-CH₂ | - | 2-tert.-Butyl-6-ethylpyrimidin-4-yl |
| A-279 | CH₂-CH₂-CH₂-CH₂ | - | 2-tert.-Butyl-6-n-propylpyrimidin-4-yl |
| A-280 | CH₂-CH₂-CH₂-CH₂ | - | 2-tert.-Butyl-6-isopropylpyrimidin-4-yl |
| A-281 | CH₂-CH₂-CH₂-CH₂ | - | 2,6-Bis(tert.-butyl)pyrimidin-4-yl |
| A-282 | CH₂-CH₂-CH₂-CH₂ | - | 2-tert.-Butyl-6-cyclopropylpyrimidin-4-yl. |
| A-283 | CH₂-CH₂-CH₂-CH₂ | - | 2-tert.-Butyl-6-cyclobutylpyrimidin-4-yl |
| A-324 | C(O)-CH₂-CH₂-CH₂ | - | 2-tert.-Butyl-6-trifluormethylpyrimidin-4-yl |
| A-325 | C(O)-CH₂-CH₂-CH₂ | - | 2-tert.-Butyl-6-difluormethylpyrimidin-4-yl |
| A-326 | C(O)-CH₂-CH₂-CH₂ | - | 2-tert.-Butyl-6-phenylpyrimidin-4-yl |
| A-327 | C(O)-CH₂-CH₂-CH₂ | - | 2-tert.-Butyl-8-methylpyrimidin-4-yl- |
| A-328 | C(O)-CH₂-CH₂-CH₂ | - | 2-tert.-Butyl-6-ethylpyrimidin-4-yl |
| A-329 | C(O)-CH₂-CH₂-CH₂ | - | 2-tert.-Butyl-6-n-propylpyrimidin-4-yl |
| A-330. | C(O)-CH₂-CH₂-CH₂ | - | 2-tert.-Butyl-6-isopropylpyrimidin-4-yl |
| A-331 | C(O)-CH₂-CH₂-CH₂ | - | 2,6-Bis(tert.-butyl)pyrimidin-4-yl |
| A-332 | C(O)-CH₂-CH₂-CH₂ | - | 2-tert.-Butyl-6-cyclopropylpyrimidin-4-yl |
| A-333 | C(O)-CH₂-CH₂-CH₂ | - | 2-*tert*.-Butyl-6-cyclobutylpyrirnidin-4-yl |
| A-334 | C(O)-CH₂-CH₂-CH₂-CH₂ | - | 2-tert.-Butyl-6-trifluormethylpyrimidin-4-yl |
| A-335 | C(O)-CH₂-CH₂-CH₂-CH₂ | - | 2-tert.-Butyl-6-difluormethylpyrimidin-4-yl |
| A-336 | C(O)-CH₂-CH₂-CH₂-CH₂ | - | 2-tert.-Butyl-6-phenylpyrimidin-4-yl |
| A-337 | C(O)-CH₂-CH₂-CH₂-CH₂ | - | 2-tert.-Butyl-6-methylpyrimidin-4-yl |
| A-338 | C(O)-CH₂-CH₂-CH₂-CH₂ | - | 2-tert.-Butyl-6-ethylpyrimidin-4-yl |
| A-339 | C(O)-CH₂-CH₂-CH₂-CH₂ | - | 2-tert.-Butyl-6-n-propylpyrimidin-4-yl |
| A-340 | C(O)-CH₂-CH₂-CH₂-CH₂ | - | 2-tert.-Butyl-6-isopropylpyrimidin-4-yl |
| A-341 | C(O)-CH₂-CH₂-CH₂-CH₂ | - | 2,6-Bis(tert.-butyl)pyrimidin-4-yl |
| A-342 | C(O)-CH₂-CH₂-CH₂-CH₂ | - | 2-tert.-Butyl-6-cyclopropylpyrimidin-4-yl |
| A-343 | C(O)-CH₂-CH₂-CH₂-CH₂ | - | 2-tert.-Butyl-6-cyclobutylpyrimidin-4-yl |
| A-344 | CH₂-CH₂-CH₂-CH₂ | - | 5-tert.-Butyl-3-trifluormethylphenyl |
| A-345 | CH₂-CH₂-CH₂-CH₂ | - | 5-tert.-Butyl-3-difluormethylphenyl |
| A-346 | CH₂-CH₂-CH₂-CH₂ | - | 5-tert.-Butyl-3-phenylphenyl |
| A-347 | CH₂-CH₂-CH₂-CH₂ | - | 3-tert.-Butyl-5-methylphenyl |
| A-348 | CH₂-CH₂-CH₂-CH₂ | - | 3-tert.-Butyl-5-ethylphenyl |
| A-349 | CH₂-CH₂-CH₂-CH₂ | - | 3-tert.-Butyl-5-n-propylphenyl |
| A-350 | CH₂-CH₂-CH₂-CH₂ | - | 3-tert.-Butyl-5-isopropylphenyl |
| A-351 | CH₂-CH₂-CH₂-CH₂ | - | 3,5-Bis(tert.-butyl)phenyl |
| A-352 | CH₂-CH₂-CH₂-CH₂ | - | 3-tert.-Butyl-5-cyclopropylphenyl |
| A-353 | CH₂-CH₂-CH₂-CH₂ | - | 3-tert.-Butyl-5-cycfobutylphenyl |
| A-394 | C(O)-CH₂-CH₂-CH₂ | - | 5-tert.-Butyl-3-trifluormethylphenyl |
| A-395 | C(O)-CH₂-CH₂-CH₂ | - | 5-tert.-Butyl-3-difiuormethylphenyl |
| A-396 | C(O)-CH₂-CH₂-CH₂ | - | 5-tert.-Butyl-3-phenylphenyl |
| A-397 | C(O)-CH₂-CH₂-CH₂ | - | 3-tert.-Butyl-5-methylphenyl |
| A-398 | C(O)-CH₂-CH₂-CH₂ | - | 3-tert.-Butyl-5-ethylphenyl |
| A-399 | C(O)-CH₂-CH₂-CH₂ | - | 3-tert.-Butyl-5-n-propylphenyl |
| A-400 | C(O)-CH₂-CH₂-CH₂ | - | 3-tert.-Butyl-5-isopropylphenyl |
| A-401 | C(O)-CH₂-CH₂-CH₂ | - | 3,5-Bis(tert.-butyl)phenyl |
| A-402 | C(O)-CH₂-CH₂-CH₂ | - | 3-tert.-Butyl-5-cyclopropylphenyl |
| A-403 | C(O)-CH₂-CH₂-CH₂ | - | 3-tert-Butyl-5-cyclobutylphenyl |
| A-404 | C(O)-CH₂-CH₂-CH₂-CH₂ | - | 5-tert.-Butyl-3-trifluormethylphenyl |
| A-405 | C(O)-CH₂-CH₂-CH₂-CH₂ | - | 5-tert.-Butyl-3-diftuarmethylphenyl |
| A-406 | C(O)-CH₂CH₂-CH₂-CH₂ | - | 5-tert.-Butyl-3-phenylphenyl |
| A-407 | C(O)-CH₂-CH₂-CH₂-CH₂ | - | 3-tert.-Butyl-5-methylphenyl |
| A-408 | C(O)-CH₂-CH₂-CH₂-CH₂ | - | 3-tert.-Butyl-5-ethylphenyl |
| A-409 | C(O)-CH₂-CH₂-CH₂-CH₂ | - | 3-tert.-Butyl-5-n-propylphenyl |
| A-410 | C(O)-CH₂-CH₂-CH₂-CH₂ | - | 3-tert.-Butyl-5-isopropylphenyl |
| A-411 | C(O)-CH₂-CH₂-CH₂-CH₂ | - | 3,5-Bis(tert.-butyl)phenyl |
| A-412 | C(O)-CH₂-CH₂-CH₂-CH₂ | - | 3-tert.-Butyl-5-cyclopropylphenyl |
| A-413 | C(O)-CH₂-CH₂CH₂-CH₂ | - | 3-tert.-Butyl-5-cyclobutylphenyl |
| A-415 | CH₂-CH₂-CH₂-CH₂ | - | 2-Methylphenyl |
| A-416 | CH₂-CH₂-CH₂-CH₂ | - | 2-Fluorphenyl |
| A-417 | CH₂-CH₂-CH₂-CH₂ | - | 2,3-Dimethylphenyl |
| A-418 | CH₂-CH₂-CH₂-CH₂ | - | 2-Methoxyphenyl |
| A-419 | CH₂-CH₂-CH₂-CH₂ | - | 2-Chlorphenyl |
| A-420 | CH₂-CH₂-CH₂-CH₂ | - | 2-Ethoxyphenyl |
| A-421 | CH₂-CH₂-CH₂-CH₂ | - | 3-Trifluormethylphenyl |
| A-422 | CH₂-CH₂-CH₂-CH₂ | - | 2,4-Dichlorphenyl |
| A-423 | CH₂-CH₂-CH₂-CH₂ | - | 3,5-Dichlorphenyl |
| A-424 | CH₂-CH₂-CH₂-CH₂ | - | 2,3-Dichlorphenyl |
| A-425 | CH₂-CH₂-CH₂-CH₂ | - | 3-Chlor-6-methoxyphenyl |
| A-426 | CH₂-CH₂-CH₂-CH₂ | - | 3,5-Dimethylphenyl |
| A-427 | CH₂-CH₂-CH₂-CH₂ | - | 2-Cyanophenyl |
| A-428 | CH₂-CH₂-CH₂-CH₂ | - | 4-Chlor-3-trifluormethylphenyl |
| A-429 | CH₂-CH₂-CH₂-CH₂ | - | 3,5-Trifluormethylphenyl |
| A-430 | CH₂-CH₂-CH₂-CH₂ | - | 2-Methylpyridin-6-yl |
| A-431 | CH₂-CH₂-CH₂-CH₂ | - | 3-Cyanopyridin-2-yl |
| A-432 | CH₂-CH₂-CH₂-CH₂ | - | 3-Cyanopyridin-6-yl |
| A-433 | CH₂-CH₂-CH₂-CH₂ | - | 3-Trifluormethylpyridin-2-yl |
| A-434 | CH₂-CH₂-CH₂-CH₂ | - | 3-Trifluormethylpyridin-6-yl |
| A-435 | CH₂-CH₂-CH₂-CH₂ | - | 3-Chlor-5-trifluormethylpyridin-2-yl |
| A-436 | CH₂-CH₂-CH₂-CH₂ | - | 3,5-Dichlorpyridin-4-yl |
| A-437 | CH₂-CH₂-CH₂-CH₂ | - | 4-Trifluorpyrimidin-2-yl |
| A-438 | CH₂-CH₂-CH₂-CH₂ | - | 5-Brompyrimidin-2-yl |
| A-439 | CH₂-CH₂-CH₂-CH₂ | - | 5-Fluorpyrimidin-2-yl |
| A-440 | CH₂-CH₂-CH₂-CH₂ | - | 2-Cyanopyridazin-3-yl |
| A-441 | CH₂-CH₂-CH₂-CH₂ | - | 5-Nitrothiadiazol-2-yl |
| A-442 | CH₂-CH₂-CH₂-CH₂ | - | 4-Methylthladiazol-2-yl |
| A-555 | C(O)-CH₂-CH₂-CH₂ | - | 2-Methylphenyl |
| A-556 | C(O)-CH₂-CH₂-CH₂ | - | 2-Fluorphenyl |
| A-557 | C(O)-CH₂-CH₂-CH₂ | - | 2,3-Dimethylphenyl |
| A-558 | C(O)-CH₂-CH₂-CH₂ | - | 2-Methoxyphenyl |
| A-559 | C(O)-CH₂-CH₂-CH₂ | - | 2-Chlorphenyl |
| A-560 | C(O)-CH₂-CH₂-CH₂ | - | 2-Ethoxyphenyl |
| A-561 | C(O)-CH₂-CH₂-CH₂ | - | 3-Trifluormethylphenyl |
| A-562 | C(O)-CH₂-CH₂-CH₂ | - | 2,4-Dichlorphenyl |
| A-563 | C(O)-CH₂-CH₂-CH₂ | - | 3,5-Dichlorphenyl |
| A-564 | C(O)-CH₂-CH₂-CH₂ | - | 2,3-Dichlorphenyl |
| A-565 | C(O)-CH₂-CH₂-CH₂ | - | 3-Chlor-6-methoxyphenyl |
| A-566 | C(O)-CH₂-CH₂-CH₂ | - | 3,5-Dimethylphenyl |
| A-567 | C(O)-CH₂-CH₂-CH₂ | - | 2-Cyanophenyl |
| A-568 | C(O)-CH₂-CH₂-CH₂ | - | 4-Chlor-3-trifluormethylphenyl |
| A-569 | C(O)-CH₂-CH₂-CH₂ | - | 3,5-Trifluormethylphenyl |
| A-570 | C(O)-CH₂-CH₂-CH₂ | - | 2-Methylpyridin-6-yl |
| A-571 | C(O)-CH₂-CH₂-CH₂ | - | 3-Cyanopyridin-2-yl |
| A-572 | C(O)-CH₂-CH₂-CH₂ | - | 3-Cyanopyridin-6-yl |
| A-573 | C(O)-CH₂-CH₂-CH₂ | - | 3-Trifluormethylpyridin-2-yl |
| A-574 | C(O)-CH₂-CH₂-CH₂ | - | 3-Trifluormethylpyridin-6-yl |
| A-575 | C(O)-CH₂-CH₂-CH₂ | - | 3-Chlor-5-trifluarmethylpyridin-2-yl |
| A-576 | C(O)-CH₂-CH₂-CH₂ | - | 3,5-Dichlorpyridin-4-yl |
| A-577 | C(O)-CH₂-CH₂-CH₂ | - | 4-Trifluorpyrimidin-2-yl |
| A-578 | C(O)-CH₂-CH₂-CH₂ | - | 5-Brompyrimidin-2-yl |
| A-579 | C(O)-CH₂-CH₂-CH₂ | - | 5-Fluorpyrimidin-2-yl |
| A-580 | C(O)-CH₂-CH₂-CH₂ | - | 2-Cyanopyridazin-3-yl |
| A-581 | C(O)-CH₂-CH₂-CH₂ | - | 5-Nitrothiadiazol-2-yl |
| A-582 | C(O)-CH₂-CH₂-CH₂ | - | 4-Methylthiadiazol-2-yl |
| A-583 | C(O)-CH₂-CH₂-CH₂-CH₂ | - | 2-Methylphenyl |
| A-584 | C(O)-CH₂-CH₂-CH₂-CH₂ | - | 2-Fluorphenyl |
| A-585 | C(O)-CH₂-CH₂-CH₂-CH₂ | - | 2,3-Dimethylphenyl |
| A-586 | C(O)-CH₂-CH₂-CH₂-CH₂ | - | 2-Methoxyphenyl |
| A-587 | C(O)-CH₂-CH₂-CH₂-CH₂ | - | 2-Chlorphenyl |
| A-588 | C(O)-CH₂-CH₂-CH₂-CH₂ | - | 2-Ethoxyphenyl |
| A-589 | C(O)-CH₂-CH₂-CH₂-CH₂ | - | 3-Trifluormethylphenyl |
| A-590 | C(O)-CH₂-CH₂-CH₂-CH₂ | - | 2,4-Dichlorphenyl |
| A-591 | C(O)-CH₂-CH₂-CH₂-CH₂ | - | 3,5-Dichlorphenyl |
| A-592 | C(O)-CH₂-CH₂-CH₂-CH₂ | - | 2,3-Dichlorphenyl |
| A-593 | C(O)-CH₂-CH₂-CH₂-CH₂ | - | 3-Chlor-6-methoxyphenyl |
| A-594 | C(O)-CH₂-CH₂-CH₂-CH₂ | - | 3,5-Dimethylphenyl |
| A-595 | C(O)-CH₂-CH₂-CH₂-CH₂ | - | 2-Cyanophenyl |
| A-596 | C(O)-CH₂-CH₂-CH₂-CH₂ | - | 4-Chlor-3-trifluormethylphenyl |
| A-597 | C(O)-CH₂-CH₂-CH₂-CH₂ | - | 3,5-Trifluormethylphenyl |
| A-598 | C(O)-CH₂-CH₂-CH₂-CH_{z} | - | 2-Methylpyridin-6-yl |
| A-599 | C(O)-CH₂-CH₂-CH₂-CH₂ | - | 3-Cyanopyridin-2-yl |
| A-600 | C(O)-CH₂-CH₂-CH₂-CH₂ | - | 3-Cyanopyridin-6-yl |
| A-601 | C(O)-CH₂-CH₂-CH₂-CH₂ | - | 3-Trifluormethylpyridin-2-yl |
| A-602 | C(O)-CH₂-CH₂-CH₂-CH₂ | - | 3-Trifluormethylpyridin-6-yl |
| A-603 | C(O)-CH₂-CH₂-CH₂-CH₂ | - | 3-Chlor-5-trifluormethylpyridin-2-yl |
| A-604 | C(O)-CH₂-CH₂-CH₂-CH₂ | - | 3,5-Dichlorpyridin-4-yl |
| A-605 | C(O)-CH₂-CH₂-CH₂-CH₂ | - | 4-Trifluorpyrimidin-2-yl |
| A-606 | C(O)-CH₂-CH₂-CH₂-CH₂ | - | 5-Brompyrimidin-2-yl |
| A-607 | C(O)-CH₂-CH₂-CH₂-CH₂ | - | 5-Fluorpyrimidin-2-yl |
| A-608 | C(O)-CH₂-CH₂-CH₂-CH₂ | - | 2-Cyanopyridazin-3-yl |
| A-609 | C(O)-CH₂-CH₂-CH₂-CH₂ | - | 5-Nitrothiadiazol-2-yl |
| A-610 | C(O)-CH₂-CH₂-CH₂-CH₂ | - | 4-Methylthiadiazol-2-yl |
| A-611 | CH₂-CH₂-CH₂-CH₂ | CH₂ | 3,4-Methylphenyl |
| A-613 | CH₂-CH₂-CH₂-CH₂ | CH₂ | 2,5-Bis(methoxy)phenyl |
| A-614 | CH₂-CH₂-CH₂-CH₂ | CH₂ | 3,5-Dichlorphenyl |
| A-615 | CH₂-CH₂-CH₂-CH₂ | CH₂ | 3-Cyanophenyl |
| A-616 | CH₂-CH₂-CH₂-CH₂ | CH₂ | 4-Cyanophenyl |
| A-617 | CH₂-CH₂-CH₂-CH₂ | CH₂ | 2-Pyridyl |
| A-618 | CH₂-CH₂-CH₂-CH₂ | CH₂ | 3-Pyridyl |
| A-619 | CH₂-CH₂-CH₂-CH₂ | CH₂ | 4-Pyridyl |
| A-620 | CH₂-CH₂-CH₂-CH₂ | CH₂ | 2,3-Dichlorphenyl |
| A-621 | CH₂-CH₂-CH₂-CH₂ | CH₂ | 2,5-Dimethylphenyl |
| A-622 | CH₂-CH₂-CH₂-CH₂ | CH₂ | 2-Methylnaphthalin-1-yl |
| A-623 | CH₂-CH₂-CH₂-CH₂ | CH₂ | 2-Thienyl |
| A-624 | CH₂-CH₂-CH₂-CH₂ | CCH₃ | Phenyl |
| A-681 | C(O)-CH₂-CH₂-CH₂ | CH₂ | 3,4-Methylphenyl |
| A-683 | C(O)-CH₂-CH₂-CH₂ | CH₂ | 2,5-Bis(methoxy)phenyl |
| A-684 | C(O)-CH₂-CH₂-CH₂ | CH₂ | 3,5-Dichlorphenyl |
| A-685 | C(O)-CH₂-CH₂-CH₂ | CH₂ | 3-Cyanophenyl |
| A-686 | C(O)-CH₂-CH₂-CH₂ | CH₂ | 4-Cyanophenyl |
| A-687 | C(O)-CH₂-CH₂-CH₂ | CH₂ | 2-Pyridyl |
| A-688 | C(O)-CH₂-CH₂-CH₂ | CH₂ | 3-Pyridyl |
| A-689 | C(O)-CH₂-CH₂-CH₂ | CH₂ | 4-Pyridyl |
| A-690 | C(O)-CH₂-CH₂-CH₂ | CH₂ | 2,3-Dichlorphenyl |
| A-691 | C(O)-CH₂-CH₂-CH₂ | CH₂ | 2,5-Dimethylphenyl |
| A-692 | C(O)-CH₂-CH₂-CH₂ | CH₂ | 2-Methylnaphthalin-1-yl |
| A-693 | C(O)-CH₂-CH₂-CH₂ | CH₂ | 2-Thienyl |
| A-694 | C(O)-CH₂-CH₂-CH₂ | CCH₃ | Phenyl |
| A-695 | C(O)-CH₂-CH₂-CH₂-CH₂ | CH₂ | 3,4-Methylphenyl |
| A-697 | C(O)-CH₂-CH₂-CH₂-CH₂ | CH₂ | 2,5-Bis(methoxy)phenyl |
| A-698 | C(O)-CH₂-CH₂-CH₂-CH₂ | CH₂ | 3,5-Dichlorphenyl |
| A-699 | C(O)-CH₂-CH₂-CH₂-CH₂ | CH₂ | 3-Cyanophenyl |
| A-700 | C(O)-CH₂-CH₂-CH₂-CH₂ | CH₂ | 4-Cyanophenyl |
| A-701 | C(O)-CH₂-CH₂-CH₂-CH₂ | CH₂ | 2-Pyridyl |
| A-702 | C(O)-CH₂-CH₂-CH₂-CH₂ | CH₂ | 3-Pyridyl |
| A-703 | C(O)-CH₂-CH₂-CH₂-CH₂ | CH₂ | 4-Pyridyl |
| A-704 | C(O)-CH₂-CH₂-CH₂-CH₂ | CH₂ | 2,3-Dichlorphenyl |
| A-705 | C(O)-CH₂-CH₂-CH₂-CH₂ | CH₂ | 2,5-Dimethylphenyl |
| A-706 | C(O)-CH₂-CH₂-CH₂-CH₂ | CH₂ | 2-Methylnaphthalin-1-yl |
| A-707 | C(O)-CH₂-CH₂-CH₂-CH₂ | CH₂ | 2-Thienyl |
| A-708 | C(O)-CH₂-CH₂-CH₂-CH₂ | CCH₃ | Phenyl |

Besonders bevorzugt sind weiterhin die Verbindungen der Formel I-Aa.b, worin die Variablen D, E und Ar die oben genannten, insbesondere die als bevorzugt genannten, Bedeutungen aufweisen. Beispiele für derartige Verbindungen sind die Verbindungen I-Aa.b.1 bis I-Aa.b.708, in denen die Variablen D, E und Ar gemeinsam die in einer Zeile der Tabelle A angegebenen Bedeutungen aufweisen.

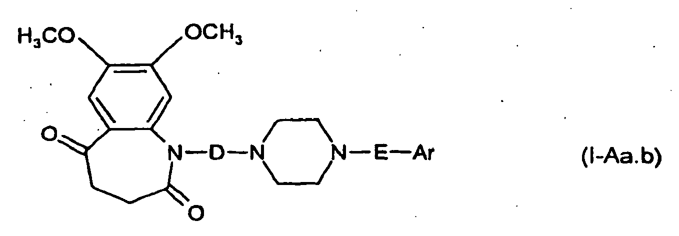

Besonders bevorzugt sind weiterhin die Verbindungen der Formel I-Aa.c. worin die Variablen D, E und Ar die oben genannten, insbesondere die als bevorzugt genannten, Bedeutungen aufweisen. Beispiele für derartige Verbindungen sind die Verbindungen 1-Aa.c.1 bis I-Aa.c.708, in denen die Variablen.D, E und Ar gemeinsam die in einer Zeile der Tabelle A angegebenen Bedeutungen aufweisen.

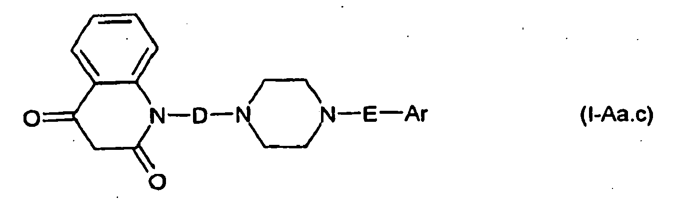

Die Herstellung der erfindungsgemäßen Verbindungen I kann in Analogie zu dem eingangs zitierten Stand der Technik sowie nach bekannten Verfahren zur Herstellung Ketolactamen erfolgen. Ein wichtiger Zugang zu den erfindungsgemäßen Verbindungen ist in Schema 1 dargestellt.

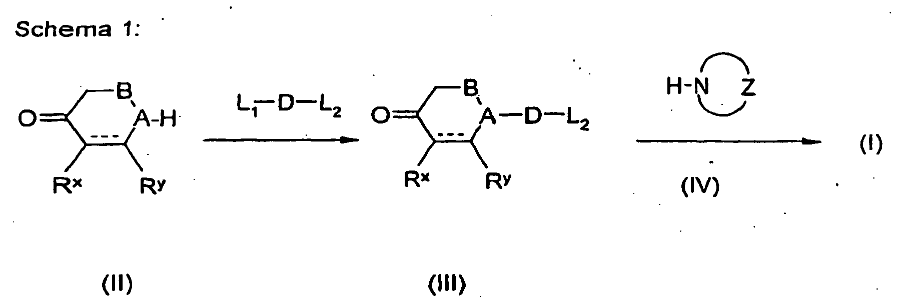

In Schema 1 haben A, B, D, R^{x}, R^{y} und NZ die zuvor genannten Bedeutungen. L₁ und L₂ stehen für nucleophil verdrängbare Abgangsgruppen. Beispiele für geeignete nucleophil verdrängbare Abgangsgruppen, sind Halogen, insbesondere Chlor, Brom oder lod, Alkyl- und Arylsulfonat wie Mesylat, Tosylat. L₁ und L₂ sind vorzugsweise voneinander verschieden und weisen eine unterschiedliche Reaktivität auf. Beispielsweise steht L₁ für Brom oder lod und L₂ für Chlor. Die für die Umsetzung erforderlichen Reaktionsbedingungen entsprechen den für nucleophile Substitutionen üblichen Reaktionsbedingungen. Sofern D für eine Gruppe C(O)Alkylen steht, dann bedeutet L₁ insbesondere Halogen und speziell Chlor.

Verbindungen der allgemeinen Formel IV sind entweder literaturbekannt, z.B. aus WO 96/02519, WO 97/25324, WO 99/02503, WO 00/42036, DE 10304870.7 oder der in diesen Schriften zitierten Literatur bekannt oder können nach den dort beschriebenen Verfahren hergestellt werden.

Die Verbindungen der Formel II sind ebenfalls bekannt und teilweise kommerziell erhältlich oder können in Analogie zu bekannten Verfahren hergestellt werden, wie beispielsweise beschrieben in: J. Am. Chem. Soc. 1958, 80, S. 2172-2178; J. Chem. Soc. 1959, S.3111; J. Chem. Soc. 1934, S. 1326; Heterocycles 1977, 8, S. 345-350; Tetrahedron Lett. 1993, 34, 5855; Arch. Pharm. 1991, 324, 579; J. Med. Chem. 1990, 33, 633; J. Med. Chem. 2000, 43, 1878; J. Org. Chem. 1972, 37, S. 2849. Monatsh. Chem. 1965, 96, 418, Synlett 2002, 8, S. 1350, Tetrahedron Lett, 1993, 34, S. 5855 und J. Photochem. 28 (1985) S. 69-70.

Die erfindungsgemäßen Verbindungen können z.T. auch nach der in Schema 2 dargestellten Synthese hergestellt werden:

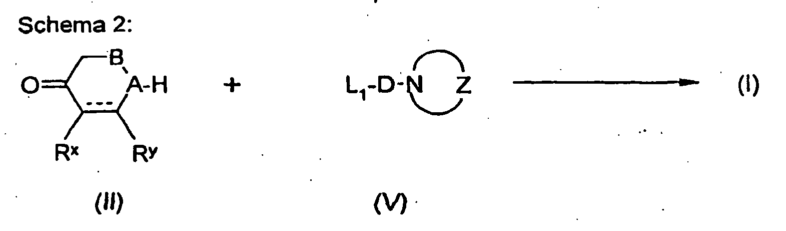

In Schema 2 haben A, B, R^{x}, R^{y} und NZ die zuvor genannten Bedeutungen. D steht für C₂-C₃-Alkylen oder eine Gruppe CO-C₂-C₁₀-Alkylen, worin CO an L₁ gebunden ist. L₁ steht für eine nucleophil verdrängbare Abgangsgruppe. Beispielsweise steht L₁ für Chlor, Brom oder lod wenn D für Alkylen steht. Die für die Umsetzung erforderlichen Reaktionsbedingungen entsprechen den für nucleophile Substitutionen üblichen Reaktionsbedingungen. Sofern D für eine Gruppe C(O)Alkylen steht, dann bedeutet L₁ insbesondere Halogen und speziell Chlor.

Verbindungen der allgemeinen Formel V sind ebenfalls literaturbekannt, z.B. aus WO 96/02519, WO 97/25324, WO 99/02503, WO 00/42036, DE 10304870.7 oder aus der in diesen Schriften zitierten Literatur bekannt oder können nach den dort beschriebenen Verfahren hergestellt werden, beispielsweise durch Umsetzung einer in Schema 1 gezeigten Verbindung der Formel IV mit einer Verbindung L₁-D-L₂, worin L und D die in Schema 1 angegebenen Bedeutungen aufweisen.

Die Herstellung der Tautomere I' kann in analoger Weise wie die hier beschriebene Herstellung der Verbindung I erfolgen. Beispielsweise kann man die Tautomere l' nach dem in Schema 1 gezeigten Syntheseweg herstellen. Weiterhin kann man Verbindungen I', worin R für Alkoxy oder eine Gruppe OC(O)R⁹ steht, aus den Verbindungen I durch Umsetzung mit einem geeigneten Alkylierungsmittel oder einem geeigneten Acylierungsmittel der Formel X'-C(O)R⁹, worin X' für Halogen und insbesondere für Chlor steht, gegebenenfalls in Gegenwart einer Hilfsbase umsetzen, beispielsweise nach den in Chem. Commun. 1998, S. 2621 oder J. Org. Chem. 1959, 24, S. 41-43 beschriebenen Methoden. Außerdem kann man die Verbindung I in ihre Tautomere I' mit R = Halogen umwandeln, in dem man Sie mit einem geeigneten Halogenierungsmittel wie PCl₃ oder POCl₃ behandelt.

Sofern nichts anderes angegeben wird, erfolgen die oben beschriebenen Umsetzungen im allgemeinen in einem Lösungsmittel bei Temperaturen zwischen Raumtemperatur und der Siedetemperatur des verwendeten Lösungsmittels. Brauchbare Lösungsmittel sind beispielsweise Ether, wie Diethylether, Diisopropylether, Methyl-tert.-butylether oder Tetrahydrofuran, Dimethylformamid, Dimethylsulfoxid, Dimethoxyethan, Toluol, Xylol, Acetonitril, Ketone, wie Aceton oder Methylethylketon, oder Alkohole, wie Methanol, Ethanol oder Butanol.

Gewünschtenfalls arbeitet man in Gegenwart einer Base zur Neutralisation von bei den Umsetzungen freiwerdenden Protonen. Geeignete Basen umfassen anorganische Basen, wie Natrium- oder Kaliumcarbonat, Natrium- oder Kaliumhydrogencarbonat, weiterhin Alkoholate wie Natriummethylat, Natriumethylat, Alkalimetallhydride wie Natriumhydrid, metallorganische Verbindungen, wie Butyllithium- oder Alkylmagnesium-Verbindungen, oder organische Stickstoffbasen, wie Triethylamin oder Pyridin. Letztere können gleichzeitig als Lösungsmittel dienen.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder durch Überführen in eine Säureadditionssalz.

Die Säureadditionssalze werden in üblicher Weise durch Mischen der freien Base mit der entsprechenden Säure, gegebenenfalls in Lösung in einem organischen Lösungsmittel, beispielsweise einem niedermolekularen Alkohol, wie Methanol, Ethanol oder Propanol, einem Ether, wie Methyl-tert-butylether, Diisopropylether, einem Keton, wie Aceton oder Methylethylketon oder einem Ester, wie Essigsäureethylester, hergestellt.

Bei den erfindungsgemäßen Verbindungen der Formel 1 handelt es sich in der Regel um hochselektive Dopamin-D₃-Rezeptor-Liganden, die auf Grund ihrer geringen Affinität gegenüber anderen Rezeptoren wie D₁-Rezeptoren, D₄-Rezeptoren, α1- und/oder α2-adrenergen-Rezeptoren , muskarinergen Rezeptoren, histaminischen Rezeptoren, Opiat-Rezeptoren und insbesondere gegenüber Dopamin-D₂-Rezeptoren, nebenwirkungsärmer als die klassischen Neuroleptika sind, bei denen es sich um D₂-Rezeptor Antagonisten handelt.

Die hohe Affinität der erfindungsgemäßen Verbindungen gegenüber D₃-Rezeptoren spiegelt sich in sehr geringen in vitro Kᵢ-Werte von in der Regel weniger als 100 nM (nmol/l) und vor allem von weniger als 50 nM wieder. Beispielsweise können Bindungsaffinitäten zu D₃-Rezeptoren in Rezeptorbindungsstudien über die Verdrängung von [¹²⁵I]-lodosulprid bestimmt werden.

Erfindungsgemäß von besonderer Bedeutung sind Verbindungen deren Selektivität Kᵢ(D₂)/Kᵢ(D₃) vorzugsweise wenigstens 10, besser noch wenigstens 30 und besonders vorteilhaft wenigstens 50 beträgt. Rezeptorbindungsstudien an D₁-, D₂- und D₄-Rezeptoren können beispielsweise über die Verdrängung von [³H]SCH23390, [¹²⁵I]lodosulprjd bzw. [¹²⁵I]Spiperon vorgenommen werden.

Die Verbindungen sind aufgrund ihres Bindungsprofils zur Behandlung von Erkrankungen brauchbar, die auf Dopamin-D₃-Liganden ansprechen, d. h. sie sind zur Behandlung von solchen Störungen bzw. Erkrankungen wirksam, bei denen eine Beeinflussung (Modulation) der Dopamin-D₃-Rezeptoren zur Verbesserung des Krankheitsbilds oder zum Ausheilen der Krankheit führt. Derartige Erkrankungen sind z. B. Störungen oder Erkrankungen des zentralen Nervensystems.

Unter Störungen bzw. Erkrankungen des zentralen Nervensystems versteht man Störungen, die Rückenmark und vor allem das Gehim betreffen. Der Begriff "Störung" im erfindungsgemäßen Sinne bezeichnet Anomalien, die in der Regel als krankhafte Zustände bzw. Funktionen angesehen werden und sich in Form bestimmter Anzeichen, Symptome und/oder Fehlfunktionen zu erkennen geben können. Die erfindungsgemäße Behandlung kann auf einzelne Störungen, d. h. Anomalien bzw. krankhafte Zustände gerichtet sein, es können aber auch mehrere, gegebenenfalls ursächlich miteinander verbundene Anomalien zu Mustern, d.h. Syndromen, zusammengefasst sein, die erfindungsgemäß behandelt werden können.

Zu den erfindungsgemäß behandelbären Störungen gehören vor allem psychiatrische und neurologische Störungen. Hierzu zählen insbesondere organische Störungen, symptomatische Störungen eingeschlossen, wie Psychosen vom akuten exogenen Reaktionstyp oder Begleit-Psychosen organischer bzw. exogener Ursache, z. B. bei Stoffwechselstörungen, Infektionen und Endokrinopathien; endogene Psychosen, wie Schizophrenie sowie schizotype und wahnhafte Störungen; affektive Störungen, wie Depressionen, Manie bzw. manisch-depressive Zustände; sowie Mischformen der zuvor geschilderten Störungen; neurotische und somatoforme Störungen sowie Störungen bei Belastung; dissoziative Störungen, z.B. Bewusstseinsausfälle, -eintrübungen und -spaltungen und Persönlichkeitsstörungen; Störungen von Aufmerksamkeit und Wach/Schlafverhalten, wie Verhaltensstörungen und emotionale Störungen, deren Beginn in der Kindheit und Jugend liegt, z.B. Hyperaktivität bei Kindern, intellektuelle Defizite, insbesondere Aufmerksamkeitsstörungen (attention deficit disorders), Gedächtnis- und kognitive Störungen, z.B. Lem- und Gedächtnisschwäche (impaired cognitive function), Demenz, Narkolepsie und Schlafstörungen, z.B. restless legs syndrome; Entwicklungsstörungen; Angstzustände; Delirium; Störungen des Sexuallebens, z.B. Impotenz des Mannes; Essstörungen, z.B. Anorexie oder Bulimie; Sucht; und weitere nicht näher bezeichnete psychiatrische Störungen.

Zu den erfindungsgemäß behandelbaren Störungen gehören auch Parkinson und Epilepsie und insbesondere die damit in Zusammenhang stehenden affektiven Störungen. Zu Suchterkrankungen gehören die durch den Missbrauch von psychotropen Substanzen, wie Arzneimittel oder Drogen, verursachte psychische Störungen und Verhaltensstörungen sowie andere Suchterkrankungen, wie beispielsweise die Spielsucht und die Impulsive-Kontrollstörung (Impulse Control Disorders not elsewhere classified). Suchterzeugende Substanzen sind beispielsweise: Opioide (z. B. Morphin, Heroin, Codein); Kokain; Nikotin; Alkohol; Substanzen, die mit dem GABA-Chlorid-Kanal-Komplex interagieren, Sedativa, Hypnotika oder Tranquilizer, beispielsweise Benzodiazepine; LSD; Cannabinoide; psychomotorische Stimulanzien, wie 3,4-Methylendioxy-N-methylamphetamin (Ecstasy); Amphetamin und amphetaminartigen Substanzen wie Methylphenidat oder sonstige Stimulanzien einschließlich Koffein. Als suchterzeugende Substanzen kommen insbesondere Opioide, Kokain, Amphetamin oder amphetaminartige Substanzen, Nikotin und Alkohol in Betracht.

Im Hinblick auf die Behandlung von Suchterkrankungen sind unter den erfindungsgemäßen Verbindungen der Formel I solche besonders bevorzugt, die selbst keine psychotrope Wirkung besitzen. Dies kann im Test auch an Ratten beobachtet werden, die nach Verabreichung erfindungsgemäß brauchbarer Verbindungen die Selbstverabreichung von psychotropen Substanzen, beispielsweise Kokain, drosseln.

Gemäß einem weiteren Aspekt der vorliegenden Erfindung sind die erfindungsgemäßen Verbindungen zur Behandlung von Störungen geeignet, deren Ursachen zumindest zum Teil auf eine anomale Aktivität von Dopamin-D₃-Rezeptoren zurückzuführen sind.

Gemäß einem anderen Aspekt der vorliegenden Erfindung richtet sich die Behandlung vor allem auf diejenigen Störungen, die sich über eine Bindung von vorzugsweise exogen zugesetzten Bindungspartnern (Liganden) an Dopamin-D₃-Rezeptoren im Sinne einer zweckmäßigen medizinischen Behandlung beeinflussen lassen.

Die mit den erfindungsgemäßen Verbindungen behandelbaren Erkrankungen sind häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten.

Durch die erfindungsgemäßen Verbindungen lassen sich eine Vielzahl von Anzeichen, Symptomen und/oder Fehlfunktionen behandeln, die mit den Störungen des zentralen Nervensystems und insbesondere den vorstehend genannten Zuständen zusammenhängen. Hierzu gehören beispielsweise ein gestörter Realitätsbezug, mangelnde Einsicht und Fähigkeit, üblichen sozialen Normen bzw. Lebensanforderungen zu genügen, Wesensveränderungen, Veränderungen der Einzeltriebe, wie Hunger, Schlaf, Durst, etc., und der Stimmungslage, Störungen der Merk- und Kombinationsfähigkeit, Persönlichkeitsveränderungen, insbesondere Affektlabilität, Halluzinationen, Ich-Störungen, Zerfahrenheit, Ambivalenz, Autismus, Depersonalisation bzw. Sinnestäuschungen, Wahnideen, skandierende Sprache, fehlende Mitbewegung, kleinschrittiger Gang, Beugehaltung von Rumpf und Gliedern, Tremor, Mimikarmut, monotone Sprache, Depressionen, Apathie, erschwerte Spontanität und Entschlusskraft, verarmte Assoziationsfähigkeit, Angst, nervöse Unruhe, Stottern, soziale Phobie, Panikstörungen, Entzugssyndrome bei Abhängigkeit, maniforme Syndrome, Erregungs- und Verwirrtheitszustände, Dysphorie, dyskinetische Syndrome und Tic-Störungen, z.B. Chorea-Huntington, Gilles-de-la-Töurette-Syndrom, Schwindelsyndrome, z.B. peripherer Lage-, Dreh- und Schwankschwindel, Melancholie, Hysterie, Hypochondrie und ähnliches. Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Vorzugsweise eignen sich die erfindungsgemäßen Verbindungen zur Behandlung von Erkrankungen des zentralen Nervensystems, insbesondere zur Behandlung von affektiven Störungen; neurotischen, Belastungs- und somatoformen Störungen und Psychosen und speziell zur Behandlung der Schizophrenie und der Depression. Aufgrund ihrer hohen Selektivität bezüglich des D₃-Rezeptors sind die erfindungsgemäßen Verbindungen I auch zur Behandlung von Nierenfunktionsstörungen, insbesondere von Nierenfunktionsstörungen, die durch Diabetes-Mellitus hervorgerufen wird (siehe WO 00/67847).

Die erfindungsgemäße Verwendung der beschriebenen Verbindungen beinhaltet im Rahmen der Behandlung ein Verfahren. Dabei wird dem zu behandelnden Individuum, vorzugsweise einem Säuger, insbesondere einem Menschen, Nutz- oder Haustier, eine wirksame Menge eines oder mehrerer Verbindungen, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren mit einbezieht.

Die Behandlung erfolgt in der Regel durch einmalige oder mehrmalige tägliche Verabfolgung, gegebenenfalls zusammen oder im Wechsel mit anderen Wirkstoffen oder wirkstoffhaltigen Präparaten, so dass einem zu behandelnden Individuum eine Tagesdosis von vorzugsweise etwa 0,1 bis 1000 mg/kg Körpergewicht bei oraler Gabe bzw. von etwa 0,1 bis 100 mg/kg Körpergewicht bei parenteraler Gabe zugeführt wird. Die Erfindung betrifft auch die Herstellung pharmazeutischer Mittel zur Behandlung eines Individuums, vorzugsweise eines Säugers, insbesondere eines Menschen, Nutz-oder Haustieres. So werden die Liganden gewöhnlich in Form von pharmazeutischen Zusammensetzungen verabreicht, die einen pharmazeutisch verträglichen Exzipienten mit wenigstens einem erfindungsgemäßen Liganden und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions-und Infusionszubereitungen, Augen- und Ohrentropfen. Auch implantierte Abgabevorrichtungen können zur Verabreichung erfindungsgemäßer Inhibitoren verwendet werden. Ferner können auch Liposomen oder Mikrosphären zur Anwendung kommen. Bei der Herstellung der Zusammensetzungen werden erfindungsgemäße Inhibitoren gewöhnlich mit einem Exzipienten vermischt oder verdünnt. Exzipienten können feste, halbfeste oder flüssige Materialien sein, die als Vehikel, Träger oder Medium für den Wirkstoff dienen.

Geeignete Exzipienten sind in den einschlägigen Arzneimonographien gelistet. Ferner können die Formulierungen pharmazeutisch akzeptable Träger oder übliche Hilfsstoffe, wie Gleitmittel; Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Die nachfolgenden Beispiele dienen zur Erläuterung der Erfindung ohne sie zu begrenzen.

Die magnetischen Kernresonanzspektraleigenschaften (NMR) beziehen sich auf chemische Verschiebungen (δ), ausgedrückt in Teile pro Million (ppm). Die relative Fläche für die Verschiebungen in dem ¹H-NMR-Spektrum entspricht der Anzahl der Wasserstoffatome für einen bestimmten funktionalen Typ in dem Molekül. Die Art der Verschiebung hinsichtlich der Multiplizität ist angegeben als Singulett (s), breites Singulett (s. br.), Dublett (d), breites Dublett (d br.), Triplett (t), breites Triplett (t br.), Quartett (q), Quintett (quint.), Multiplett (m).

### A) Herstellungsbeispiele:

### Beispiel 1 (nicht erfindungsgemäß)

### 2-(3-{4-[2-tert-Butyl-6-(trifluormethyl)pyrimidin-4-yl]piperazin-1-yl}propyl)-3,4-dihydro-1 H-2-benzazepin-1,5(2H)-dion

Zu einer Suspension von Natriumhydrid (3,40 mmol, 0,13 g, 60%, entölt) in Dimethylformamid (10 ml) gab man bei 10°C 3,4-Dihydro-1*H*-2-benzazepin-1,5(2*H*)-dion (2,85 mmol, 0,50 g; hergestellt nach Tetrahedron Lett. 1993, 34, 5855) in Dimethylformamid (5 ml) und rührte 1h bei Raumtemperatur. Anschließend wurde 2-*tert*-Butyl-4-[4-(3-chlorpropyl)piperazin-1-ylj-6-(trifluormethyl)pyrimidin (3,00 mmol, 1,09 g; hergestellt nach WO 99/02503) in Dimethylformamid (5 ml) zugetropft. Das Reaktionsgemisch wurde bei Raumtemperatur 12h nachgerührt. Das nach dem Eindampfen verbliebene Öl wurde in einem 1:1 Gemisch aus Ethylacetat und Wasser aufgenommen, extrahiert und mit einer gesättigten, wässrigen Kochsalzlösung gewaschen. Die organische Phase wurde über Natriumsulfat getrocknet und eingedampft. Der Rückstand wurde durch Chromatographie an Kieselgel (Eluierungsmittel: Dichlormethan:Methanol 95:5 v/v) gereinigt.

Als zweite Fraktion erhielt man die Titelverbindung, 2-(3-{4-[2-*tert*-Butyl-6-(trifluormethyl)pyrimidin-4-yl]piperazin-1-yl}propyl)-3,4-dihydro-1*H*-2-benzazepin-1,5(2*H*)-dion in einer Ausbeute von 20 mg.

ESI-MS: [M+Na⁺] = 526,2, 505,2, [M+H⁺] = 504,2, 252,6;
¹H-NMR (400 MHz, CDCl₃) δ (ppm): 7,90 (1H, d), 7,68-7,54 (3H, m), 6,57 (1H, s), 3,77-3,65 (8H, m), 2,99 (2H, m sym.), 2,53 (4H, t), 2,47 (2H, t), 1,89 (2H, quint.), 1,33 (9H, s).

### Beispiel 2

### 1-(3-{4-[2-tert-Butyl-6-(trifluormethyl)pyrimidin-4-yl]piperazin-1-yl}propyl)-3,4-dihydro-1H-1-benzazepin-2,5-dion

Analog zu Beispiel 1 erhielt man aus 3,4-Dihydro-1*H*-1-benzazepin-2,5-dion (2,85 mmol, 0,50 g; Herstellung nach Arch. Pharm. 1991, 324, 579) 30,0 mg der Titelverbindung.

¹H-NMR (400 MHz, CDCl₃) δ (ppm): 7,59-7,53 (2H, m), 7,33-7,24 (2H, m), 6,53 (1H, s), 3,96 (2H, s br.), 3,59 (4H, s br.), 3,02-2,92 (2H, m), 2,81 (2H, t), 2,36 (4H, t), 2,28 (2H, t), 1,73 (2H, quint.), 1,32 (9H, s).

### Beispiel 3:

### 1-(4-{4-[2-tert-Butyl-6-(trifluormethyl)pyrimidin-4-yl]piperazin-1-yl}butyl)-3,4-dihydro-1H-1-benzazepin-2,5-dion

### a) 1-(4-Chlorbutyl)-3,4-dihydro-1H-1-benzazepin-2,5-dion

Analog zu der in Beispiel 1 beschriebenen Vorgehensweise erhielt man durch Umsetzung von 3,4-Dihydro-1*H*-1-benzazepin-2,5-dion (11,42 mmol, 2,00 g) und Brom-4-chlorbutan (13,7 mmol, 2,35 g) 1,78 g der Titelverbindung, verunreinigt mit Edukt zu 30%. Das so erhaltene Gemisch wurde ohne weitere Reinigung umgesetzt.

### b) 1-(4-{4-[2-tert-Butyl-6-(trifluormethyl)pyrimidin-4-yl)piperazin-1-yl}butyl)-3,4-dihydro-1 H-1-benzazepin-2,5-dion

Man rührte 1-(4-Chlorbutyl)-3,4-dihydro-1*H*-1-benzazepin-2,5-dion (3,29 mmol, 1,75 g,70%), 2-*tert*-Butyl-4-piperazin-1-yl-6-(trifluormethyl)pyrimidin (3,56 mmol, 1,03 g; Herstellung nach WO 99/02503) und Triethylamin (13,17 mmol, 1,33 g) in Dimethylformamid (100 ml) 12h bei 100°C. Danach gab man Essigsäureethylester zu und wusch das Gemisch zweimal mit Wasser. Man trocknete die vereinigten organischen Phasen über Na₂SO₄, filtrierte und engte im Vakuum ein. Man reinigte den öligen Rückstand durch Chromatographie an Kieselgel (Eluierungsmittel: Dichlormethan:Methanol 95:5 vlv); Ausbeute 0,42 g.

ESI-MS: 519,2, [M+H⁺] = 518,2, 259,6;
¹H-NMR (500 MHz, CDCl₃) δ (ppm): 7,57-7,52 (2H, m), 7,29 (1H, t), 7,25 (1H, d+CHCl₃). 6,57 (1H, s), 3,92 (2H, s br.), 3,63 (4H, s br.), 3,01-2,95 (2H, m), 2,80 (2H, t), 2,41 (4H, t), 2,28 (2H, t), 1,53 (2H, quint.), 1,42 (2H, quint.), 1,34 (9H, s).

### Beispiel 4: (nicht erfindungsgemäß)

### 1-((2E)-4-{4-[2-tert-Butyl-6-(trifluormethyl)pyrimidin-4-yl]piperazin-1-yl}but-2-enyl)-3,4-dihydro-1H-1-benzazepin-2,5-dion

Analog Beispiel 1 erhielt man aus 3,4-Dihydro-1*H-*1-benzazepin-2,5-dion (4,42 mmol. 0,78 g; Darstellung nach Arch. Pharm. 1991, 324, 579) und 2-*tert*-Butyl-4-{4-[(2*E*)-4-chlorbut-2-en-1-yl]piperazin-1-yl}-6-(trifluormethyl)pyrimidin (4,64 mmol, 1,75 g, Darstellung nach WO 99/02503) 0,78 g der Titelverbindung.

ESI-MS: 517,3, [M+H⁺] = 516.3, 258,6;
¹H-NMR (500 MHz, COCl₃) δ (ppm): 7,53 (1H, d), 7,48 (1H, t), 7,27-7,21 (m+CHCl₃), 6,56 (1 H, s), 5,60 (2H, m sym.), 4,45 (2H, m), 3,64 (4H, s br.), 3,02-2,94 (4H, m), 2,84 (2H, t), 2,35 (4H, t), 1,35 (9H, s).

### Beispiel 5: (nicht erfindungsgemäß)

### 2-(4-{4-[2-tert-Butyl-6-(trifluormethyl)pyrimidin-4-yl]piperazin-1-yl}butyl)-3,4-dihydro-1H-2-benzazepin-1,5(2H)-dion

### a) 2-(4-Chlorbutyl)-3,4-dihydro-1H-2-benzazepin-1,5(2H)-dion

Analog zu Beispiel 3a erhielt man aus 3,4-Dihydro-1*H*-2-benzazepin-1,5(2*H*)-dion und Brom-4-chlorbutan (13,7 mmol, 2,35 g) 1,04 g der Titelverbindung verunreinigt mit Edukt zu 50%. Die Substanz wurde ohne weitere Reinigung umgesetzt.

### b) 2-(4-{4-[2-tert-Butyl-6-(trifluormethyl)pyrimidin-4-yl]piperazin-1-yl}butyl)-3,4-dihydro-1 H-2-benzazepin-1,5(2H)-dion

Die Herstellung erfolgte analog zu Beispiel 3b. Aus 2-(4-Chlorbutyl)-3,4-dihydro-1*H*-2-benzazepin-1,5(2*H*)-dion (1,88 mmol, 1,00 g) erhielt man 0,15 g der Titelverbindung.

ESI-MS:[M+Na⁺] = 540,3, 519,3, [M+H⁺] = 518,3, 259,6.

### Beispiel 6:

### 1-(4-{4-[2-tert-Butyl-6-(trifluormethyl)pyrimidin-4-yl]piperazin-1-yl}butyl)-7,8-dimethoxy-3,4-dihydro-1H-1-benzazepin-2,5-dion

### a) 1-(4-Chlorbutyl)-7,8-dimethoxy-3,4-dihydro-1H-1-benzazepin-2,5-dion

Analog zu Beispiel 1 erhielt man durch Umsetzung von 7,8-Dimethoxy-3,4-dihydro-1 *H-*1-benzazepin-2,5-dion (1,70 mmol, 0,40 g, Darstellung nach Arch. Pharm. 1991, 324, 579) und Brom-4-chlorbutan (2,04 mmol, 0,35 g) 0,20 g der verunreinigten Titelverbindung. Die Verbindung wird ohne Reinigung weiter angesetzt.

### b) 1-(4-{4-[2-tert-Butyl-6-(trifluormethyl)pyrimidin-4-yl]piperazin-1-yl}butyl)-7,8-dimethoxy-3,4-dihydro-1H-1-benzazepin-2,5-dion

Durch Umsetzung von 1-(4-Chlorbutyl)-7,8-dimethoxy-3,4-dihydro-1*H*-1-benzazepin-2,5-dion (0,49 mmol, 0,20 g) analog Beispiel 3b erhielt man 0,12 g der Titelverbindung.

ESI-MS: 579,2, [M+H⁺] = 578,3;

¹H-NMR (500 MHz, CDCl₃) δ (ppm): 7,09 (1H, s), 6,68 (1H, s), 6,56 (1H, s), 3,94 (3H, s), 3,92 (3H, s), 3,65 (4H, s br.), 2,97-2,92 (2H, m), 2,79 (2H, m br.), 2,40 (4H, t), 2,29 (2H, t), 1,49 (2H, quint.), 1,41 (2H, quint.), 1,31 (9H, s).

### Beispiel 7:

### 1-{4-[4-(2-tert-Butyl-6-propylpyrimidin-4-yl)piperazin-1-yl]butyl}-3,4-dihydro-1H-1-benzazepin-2,5-dion Hydrochlorid

Analog zu Beispiel 3b erhielt man durch Umsetzung von 2-*tert*-Butyl-4-piperazin-1-yl-6-propylpyrimidin (2,68 mmol, 0,70 g) mit 1-(4-Chlorbutyl)-3,4-dihydro-1*H*-1-benzazepin-2,5-dion die freie Base der Titelverbindungen. Anschließende Umsetzung der freien Base mit HCl lieferte 0,39 g der Titelverbindung als Hydrochlorid.

ESI-MS: 493,5, [M+H⁺] = 492,5, 246,7;
¹H-NMR (400 MHz, CDCl₃) δ (ppm): 7,56-7,49 (3H, m), 7,31-7,18 (2H+CHCl₃, m), 6,10 (1H, s), 3,92 (2H, t br.), 3,58 (4H, s br.), 3,02-2,94 (2H, m), 2,81 (2H, t), 2,53 (2H, t), 2,40 (4H, s br.), 2,28 (2H, t), 1,72 (1H, q), 1,51 (2H, quint.), 1,43 (2H, quint.), 1,33 (9H, s), 0,92 (1H, t).

### Beispiel 8:

### 1-{4-[4-(2-tert-Butyl-6-cyclobutylpyrimidin-4-yl)piperazin-1-yl]butyl}-3,4-dihydro-1H-1-benzazepin-2,5-dion-Hydrochlorid

Analog zu Beispiel 3b erhielt man durch Umsetzung von 2-*tert*-Butyl-4-cyclobutyl-6-piperazin-1-ylpyrimidin (1,97 mmol, 0,54 g) mit 1-(4-Chlorbutyl)-3,4-dihydro-1*H*-1-benzazepin-2,5-dion 0,39 g der Titelverbindung.

ESI-MS: [M+H⁺] = 504,5, 252,9.

### Beispiel 9:

### 1-{4-[4-(2-tert-Butyl-6-methylpyrimidin-4-yl)piperazin-1-yl]butyl}-3,4-dihydro-1H-1-benzazepin-2,5-dion Hydrochlorid

Analog zu Beispiel 3b erhielt man durch Umsetzung von 2-*tert*-Butyl-4-methyl-6-piperazin-1-ylpyrimidin (1,97 mmol, 0,46 g) mit 1-(4-Chlorbutyl)-3,4-dihydro-1*H*-1-benzazepin-2,5-dion 0,31 g der Titelverbindung.

ESI-MS: [M+H⁺] = 464,4, 232,6.

### Beispiel 10:

### 1-{4-[4-(2,6-Di-tert-butylpyrimidin-4-yl)piperazin-1-yl]butyl}-3,4-dihydro-1H-1-benzazepin-2,5-dion Hydrochlorid

Analog zu Beispiel 3b erhielt man durch Umsetzung von 2,4-Di-*tert*-butyl-6-piperazin-1-ylpyrimidin (1,26 mmol, 0,35 g) mit 1-(4-Chlorbutyl)-3,4-dihydro-1*H*-1-benzazepin-2,5-dion 0,04 g der Titelverbindung.

ESI-MS: [M+H⁺] = 506,4, 233,8

### Beispiel 11:

### 1-{4-[4-(2-tert-Butyl-6-isopropylpyrimidin-4-yl)piperazin-1-yl]butyl}-3,4-dihydro-1H-1-benzazepin-2,5-dion

Analog zu Beispiel 3b erhielt man durch Umsetzung von *2-tert-*Butyl-4-isopropyl-6-piperazin-1-ylpyrimidin (0,95 mmol, 0,25 g) mit 1-(4-Chlorbutyl)-3,4-dihydro-1*H*-1-benzazepin-2,5-dion 0,04 g der Titelverbindung.

ESI-MS: [M+H⁺] = 492,5, 246,7.

### Beispiel 12: (nicht erfindungsgemäß)

### 1-(5-{4-[2-tert-Butyl-6-(trifluormethyl)pyrimidin-4-yl]piperazin-1-yl}pentanoyl)-1,2,3,4-tetrahydro-5H-1-benzazepin-5-on

### a) 1-(5-Chlorpentanoyl)-1,2,3,4-tetrahydro-5H-1-benzazepin-5-on

Zu einer Suspension aus 1,2,3,4-Tetrahydro-5*H*-1-benzazepin-5-on (12,41 mmol, 2,00 g, Herstellung nach J. Org. Chem 1972, 37, 2849) und Kaliumcarbonat (14,89 mmol, 2,06 g) in Dimethylformamid (40mL) tropfte man bei 10 °C 5-Chlorvaleriansäurechlorid (18,61 mmol, 2,89 g) in Dimethylformamid (20 mL). Die Reaktionsmischung rührte man zunächst 1 h bei 10 °C und anschließend 3 h unter Rückfluss nach. Die ausgefallenen Salze filtrierte man ab und engte das Filtrat bis zur Trockne ein. Zu dem auf diese Weise erhaltenen Öl gab man CH₂Cl₂ und wusch das Gemisch dreimal mit jeweils 50 ml einer 5 %igen, wässrigen Natriumhydrogencarbonat-Lösung, neutralisierte mit 0, 1 N HCl (20 ml) und wusch danach dreimal mit einer gesättigten Kochsalzlösung. Man trocknete die organische Phase über Na₂SO₄ und engte im Vakuum ein; Ausbeute 3,90 g (80 % rein).

ESI-MS: [M+H+] = 280,1;

### b) 1-(5-{4-[2-tert-Butyl-6-(trifluormethyl)pyrimidin-4-yl]piperazin-1-yl}pentanoyl)-1,2,3,4-tetrahydro-5H-1-benzazepin-5-on

Man erhitzte 1-(5-Chlorpentanoyl)-1,2,3,4-tetrahydro-5*H*-1-benzazepin-5-on aus Beispiel 12a (1,43 mmol, 0,50 g), 2-tert-Butyl-4-piperazin-1-yl-6-(trifluormethyl)pyrimidin (1,43 mmol, 0,41g, Herstellung nach DE 19735410), NaBr (7,14 mmol, 0,74 g), DIPEA (Diisopropylethylamin) (14,01 mmol, 1,81 g) und N-Methylpyrrolidinon (0,6 ml) 5 h auf 120 °C. Anschließend filtrierte man das Reaktionsgemisch und engte das erhaltene Filtrat bis zur Tockne ein. Danach gab man Essigsäureethylester zu dem erhaltenen Rückstand und wusch mit gesättigter, wässriger Kochsalzlösung. Die organische Phase wurde getrocknet und danach bis zur Trockne eingeengt. Den Rückstand reinigte man durch Chromatographie an Kieselgel, Eluierungsmittel: Methyl-tert.-butylether/Methanol (0-100%); wobei man 0,31 g der Titelverbindung erhielt.

ESI-MS: [M+H⁺] = 532,7, 267,0.

### Beispiel 13: ( nicht erfindungsgemäß)

### 1-{5-[4-(2-tert Butyl-6-propylpyrimidin-4-yl)piperazin-1-yl]pentanöyl}-1,2,3,4-tetrahydro-5H-1-benzazepin-5-on Hydrochlorid

Analog zur Vorschrift zu Beispiel 12b erhielt man aus 1-(5-Chlorpentanoyl)-1,2,3,4-tetrahydro-5*H-*1-benzazepin-5-on (1,61 mmol, 0,50 g), 2-tert-Butyl-4-piperazin-1-yl-6-propylpyrimidin (1,61 mmol, 0,42 g, Herstellung nach DE 19735410) 0,40 g der Titelverbindung.

ESI-MS: [M+H⁺] = 506,4, 253,6.

### Beispiel 14: (nicht erfindungsgemäβ)

### 1-(4-{4-[2-tert-Butyl-6-(trifluormethyl)pyrimidin-4-yl]piperazin-1-yl}butanoyl)-1,2,3,4-tetrahydro-5H-1-benzazepin-5-on

### a) 1-(4-Chlorbutyryl)-1,2,3,4-tetrahydro-benzo[b]azepin-5-on

Analog zur Vorschrift zu Beispiel 12a erhielt man aus 1,2,3,4-Tetrahydro-5*H*-1-benzazepin-5-on (1,24 mmol, 0,20 g, Herstellung nach J. Org. Chem 1972, 37, 2849) und 4-Chlorbuttersäurechlorid (1,86 mmol, 0,27 g) in Dioxan (10 ml) 0,24 g 1-(4-Chlorbutyryl)-1,2,3,4-tetrahydrobenzo[b]azepin-5-on.

### b) 1-(4-{4-[2-tert-Butyl-6-(trifluormethyl)pyrimidin-4-yl]piperazin-1-yl)butanoyl)-1,2,3,4-tetrahydro-5H-1-benzazepin-5-on

Analog zur Vorschrift zu Beispiel 12b erhielt man aus 1-(4-Chlorbutyryl)-1,2,3,4-tetrahydrobenzo[b]azepin-5-on (0,45 mmol, 0,12 g) aus Beispiel 14a, 2-tert-Butyl-4-piperazin-1-yl-6-(trifluormethyl)pyrimidin (0,45 mmol, 0,12, hergestellt nach DE 19735410) 0,40 g der Titelverbindung.

ESI-MS: [M+H⁺] = 518,3, 259,6.

### Beispiel 15: (nicht erfindungsgemäß)

### 1-{4-[4-(2-tert-Butyl-6-propyl-pyrimidin-4-yl)-piperazin-1-yl]-butyryly-1,2,3,4-tetrahydro-benzo[b]azepin-5-on

Analog zur Vorschrift zu Beispiel 12b erhielt man aus 1-(4-Chlorbutyryl)-1,2,3,4-tetrahydro-benzo[b]azepin-5-on aus Beispiel 14a (0,45 mmol, 0,12 g), 2-tert-Butyl-4-piperazin-1-yl-6-propylpyrimidin (0,45 mmol, 0,12 g, hergestellt nach DE 19735410) 85,0 mg der Titelverbindung.

ESI-MS: [M+H⁺] = 492,4, 246,7.

### Beispiel 16:

### 1-{4-[4-(2-tert-Butyl-6-cyclopropyl-pyrimidin-4-yl)-piperazin-1-yl]-butyl}-3,4-dihydro-1 H-benzo[b]azepin-2,5-dion

Analog zur Vorschrift zu Beispiel 12b erhielt man aus 1-(4-Chlorbutyl)-3,4-dihydro-1*H-*1-benzazepin-2,5-dion aus Beispiel 3a (0,38 mmol, 0,10 g) und 2-tert-Butyl-4-cyclopropyl-6-piperazin-1-yl-pyrimidin (0,40 mmol, 0,05 g; hergestellt nach DE 19728996) 45,0 mg der Titelverbindung.

ESI-MS: [M+H⁺] = 490,4, 245,7.

### Beispiel 17:

### 1-{4-[4-(2-tert-Butyl-6-trifluormethyl-pyrimidin-4-yl)-piperazin-1-yl]-butyl}-1H-chinolin-2,4-dion Trifluoracetat

### a) 1-(4-Chlorbutyl)-4-hydroxy-1H-chinolin-2-on

Analog zu der in Beispiel I beschriebenen Vorgehensweise erhielt man durch Umsetzung von 4-Hydroxy-1H-chinolin-2-on (24,82 mmol, 4,00 g, hergestellt nach Monatsh. Chem. 1965, 96, 418) und Brom-4-chlorbutan (29,78 mmol, 5,11 g) 6,70 g der Titelverbindung, die mit Edukt verunreinigt ist. Das so erhaltene Gemisch wurde ohne weitere Reinigung im nächsten Schritt eingesetzt.

### b) 1-{4-[4-(2-tert-Butyl-6-trifluormethyl-pyrimidin-4-yl)-piperazin-1-yl]-butyl}-1H-chinolin-2,4-dion

Analog zur Vorschrift zu Beispiel 3b erhielt man aus 1-(4-Chlorbutyl)-4-hydroxy-1 H-chinolin-2-on aus Beispiel 17a (4,00 g) 3,20 g der Titelverbindung.

ESI-MS: [M+Na+] = 526,5, 505,5, [M+H+] = 504,5, 454,5, 252,7;
1 H-NMR (500 MHz, CDCI3) δ (ppm): 11,75 (1H, s br.), 7,85 (1H, d), 7,42 (t, 1 H), 7,26-7,20 (2H+CHCl3, m), 6,66 (1H, s.), 5,98 (1H, s); 4,18 (2H, m sym.), 3,29 (2H, m sym.), 2,21 (2H, quint.), 2,04 (2H, quint.), 1,33 (9H, s).

### Beispiel 42:

### Trifluoracetat-4-benzyl-1-[4-(2,5-dioxo-2,3,4,5-tetrahydro-benzo[b]azepin-1-yl)-butyl]-piperazin-1-ium

ESI-MS: [M+H⁺] = 406,3.

### Beispiel 43:

### Trifluoracetat-1-[4-(2,5-dioxo-2,3,4,5-tetrahydro-benzo[blazepin-1-yl)-butyl]-4-(2-pyrrol-1-yl-ethyl)-piperazin-1-ium

ESI-MS: [M+H⁺] = 409,2.

### Beispiel 44:

### Trifluoracetat-1-[4-(2,5-dioxo-2,3,4,5-tetrahydro-benzo[b]azepin-1-yl)-butyl]-4-(2-imidazol-1-yl-ethyl)-piperazin-1-ium

ESI-MS: [M+Na⁺] = 432,0, [M+H⁺] = 410,0, 342,0, 113,0.

### Beispiel 45:

### Trifluoracetat-1-[4-(2,5-dioxo-2,3,4,5-tetrahydro-benzo[b]azepin-1-yl)-butyl]-4-(2-thiophen-2-yl-ethyl)-piperazin-1-ium

ESI-MS: [M+H⁺] = 426,4.

### Beispiel 83:

### 1-{4-[4-(2,3-Dichlorphenyl)-piperazin-1-yl]-butyl}-3,4-dihydro-1H-benzo[b]azepin-2,5-dion

Analog zu Beispiel 3b erhielt man durch Umsetzung von 1-(4-Chlorbutyl)-3,4-dihydro-1 H-benzo[b]azepin-2,5-dion mit 1-(2,3-Dichlorphenyl)piperazin die Titelverbindung.

ESI-MS: 462,4,[M+H⁺] = 461,4, 460,4;
¹H-NMR (400 MHz, DMSO) δ (ppm): 7,62 (1H, .t), 7,51-7,45 (2H, m), 7,34 (1H, t), 7,28 (2H, m), 7,18-7,05 (1H, m), 3.88 (2H, t), 2.91 (6H, m), 2,66 (2H, m), 2,39 (4H, s br.), 2,19 (2H, t), 1,36 (2H, quint.), 1,26 (2H, quint.).

### Beispiel 84:

### 4-(2,4-Dichlorbenzyl)-1-[4-(2,5-dioxo-2,3,4,5-tetrahydro-benzo[b]azepin-1-yl)-butyl]-piperazinium als Furnarat

Analog zu Beispiel 3b erhielt man durch Umsetzung von 1-(4-Chlorbutyl)-3,4-dihydro-1H-benzo[b]azepin-2,5-dion mit 1-(2,4-Dichlorbenzyl)piperazin die Titelverbindung.

ESI-MS: 476,1,[M+H⁺] =475,1, 474,1, 237,6;

### Beispiel 85: (nicht erfindungsgemäß)

### 1-{4-[4-(2-tert-Butyl-6-trifluormethyl-pyrimidin-4-yl)-piperazin-1-yl]-butyl}-azepan-2,5-dion

### a) 1-(4-Chlorbutyl)-azepan-2,5-dion

Analog zu Beispiel 3a erhielt man aus Azepan-2,5-dion (2,36 mmol, 0,30 g, Darstellung nach J. Photochem. 28 (1985), 569-570) und Brom-4-chlorbutan (2,83 mmol, 0,49 g) 0,17 g der verunreinigten Titelverbindung. Die Verbindung wird ohne Reinigung weiter umgesetzt.

### b) 1-{4-[4-(2-tert-Butyl-6-trifluormethyl-pyrimidin-4-yl)-piperazin-1-yl]-butyl}-azepan-2,5-dion

Analog zu Beispiel 3b erhielt man aus 2-tert-Butyl-4-piperazin-1-yl-6-trifluormethyl-pyrimidin (0,59 mmol, 0,17 g) und 1-(4-Chlorbutyl)-azepan-2,5-dion (0,62 mmol, 0,17 g) 0,04 g der Titelverbindung.

ESI-MS: [M+H⁺] = 470,2, 235,6;
¹H-NMR (400 MHz, CDCl₃) δ (ppm): 6,57 (1H, s), 3,71 (4H, s br.), 3,60-3,46 (4H, m), 2,73-2,57 (6H, m), 2,49 (4H, s br.), 2,41 (2H, s br.), 1,33 (9H, s).

### Beispiel 86:

### 1-{4-(4-(3,5-Dichlorphenyl)-2,5-piperazin-1-yl]-butyl)-3,4-dihydro-1H-benzo[b]azepin-2,5-dion Fumarat

In Analogie zu Beispiel 3b erhielt man durch Umsetzung von 1-(4-Chlorbutyt)-3,4-dihydro-1H-benzo[b]azepin-2,5-dion mit 1-(3,5-Dichlorphenyl)-piperazin die Titelverbindung.

ESI-MS: 462,5,[M+H⁺] = 461,5, 460,5;
¹H-NMR (400 MHz, DMSO) δ (ppm): 7,62 (1H, t), 7,47 (2H, t), 7.35 (1H, t), 6,90 (2H, s), 6,85 (1H, s), 3,88 (2H, m), 3,15 (4H, m), 2,92 (2H, t), 2,67 (2H, t), 2,36 (4H, m), 2,20 (2H, t), 1,35 (2H, quint.), 1,28 (2H, quint.).

### Beispiel 87:

### 1-{4-[4-(3,5-Bis-trifluoromethyl-phenyl)-piperazin-1-yl]-butyl}-3,4-dihydro-1H-benzo[b]azepin-2,5-dion Fumarat

In Analogie zu Beispiel 3b erhielt man durch Umsetzung von 1-(4-Chlorbutyl)-3,4-dihydro-1H-benzo[b]azepin-2,5-dion mit 1-(3,5-Bistrifluormethylphenyl)-piperazin die Titelverbindung.

ESI-MS: [M+H⁺] = 528,55;
¹H-NMR (400 MHz, DMSO) δ (ppm): 7,64 (1H, t), 7,54-7,41 (4H, m), 7,34 (1H, t), 7,27 (1H, s), 3,88 (2H, m), 3,30 (4H, m br,), 2,91 (2H, t), 2,65 (2H, t), 2,40 (4H, s br,), 2,23 (2H, t), 1,36 (2H, quint.), 1,30 (2H, quint.),

### B) Beispiele für galenische Applikationsformen

### Tabletten:

Auf einer Tablettenpresse werden in üblicher Weise Tabletten folgender Zusammensetzung gepresst:
- 40 mg: Substanz des Beispiels 2
- 120 mg: Maisstärke
- 13,5 mg: Gelatine
- 45 mg: Milchzucker
- 2,25 mg: Aerosil® (chemisch reine Kieselsäure in submikroskopisch feiner Verteilung)
- 6,75 mg: Kartoffelstärke (als 6 %iger Kleister)

### Dragees:

- 20 mg: Substanz des Beispiels 2
- 60 mg: Kernmasse
- 70 mg: Verzuckerungsmasse

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### C) Biologische Untersuchungen - Rezeptorbindungsstudien:

Die zu testende Substanz wurde entweder in Methanol/Chremophor® (BASF-AG) oder in Dimethylsulfoxid gelost und anschließend mit Wasser auf die gewünschte Konzentration verdünnt.

### I. Dopamin-D₃-Rezeptor:

Der Ansatz (0,250 ml) setzte sich zusammen aus Membranen von ~ 10⁶ HEK-293 Zellen mit stabil exprimierten humanen Dopamin-D₃-Rezeptoren, 0,1 nM [¹²⁵I]-Jodosulprid und Inkubationspuffer (totale Bindung) oder zusätzlich Testsubstanz (Hemmkurve) oder 1µM Spiperon (unspezifische Bindung). Dreifach-Ansätze wurden durchgeführt.

Der Inkubationspuffer enthielt 50 mM Tris, 120 mM NaCl, 5 mM KCI, 2 mM CaCl₂, 2 mM MgCl₂ und 0,1% Rinderserumalbumin, 10 µM Quinolone, 0,1 % Ascorbinsäure (täglich frisch hergestellt). Der Puffer wurde mit HCl auf pH 7,4 eingestellt.

### II. Dopamin-D_{2L}-Rezeptor:

Der Ansatz (1 ml) setzte sich zusammen aus Membranen von - 10⁶ HEK-293 Zellen mit stabil exprimierten humanen Dopamin-D_{2L} Rezeptoren (lange Isoform) sowie 0,01 nM [¹²⁵I]-Jodospiperon und Inkubationspuffer (totale Bindung) oder zusätzlich Testsubstanz (Hemmkurve) oder 1 µM Haloperidol (unspezifische Bindung). Dreifach-Ansätze wurden durchgeführt.

Der Inkubationspuffer enthielt 50 mM Tris, 120 mM NaCl, 5 mM KCI, 2 mM CaCl₂, 2 mM MgCl₂ und 0,1 % Rinderserumalbumin. Der Puffer wurde mit HCl auf pH 7,4 eingestellt.

### III. Messung und Auswertung:

Nach Inkubation für 60 Minuten bei 25 °C wurden die Ansätze mit einem Zellsammelgerät über Wathman GF/B Glasfaserfilter unter Vakuum filtriert. Die Filter wurden mit einem Filter-Transfer-System in Szintillationsgläser überführt. Nach Zugabe von 4 ml Ultima Gold^{®} (Packard) wurden die Proben eine Stunde geschüttelt und anschließend die Radioaktivität im Beta-Counter (Packard, Tricarb 2000 oder 2200CA) gezählt. Die cp-Werte wurden anhand einer Standard-Quenchreihe mit Hilfe des geräteeigenen Programms in dpm umgerechnet.

Die Auswertung der Hemmkurven erfolgte durch iterative nichtlineare Regressionsanalyse mit dem Statistical Analysis System (SAS), ähnlich dem von Munson und Rodbard beschriebenen Programm "LIGAND".

Die erfindungsgemäßen Verbindungen zeigen in diesen Tests sehr gute Affinitäten am D₃-Rezeptor (< 100 nM häufig < 50 nM) und binden selektiv an den D₃-Rezeptor.

Die Ergebnisse der Bindungstests sind in Tabelle 1 angegeben.

**Tabelle 1:**

| Beispiel | K₁ (D₃) [nM] | Selektivität vs. D₂L |
|---|---|---|
| 3 | 0,83 | 296 |
| 4 ≠ | 1,74 | 155 |
| 6 | 4,50 | 104 |
| 7 | 1,33 | 118 |
| 10 | 1,24 | 74 |
| 16 | 0,96 | 62 |
| 86 | 2,0 | 56 |
| 87 | 7,4 | 129 |

| | | |
|---|---|---|
| K_{I}(D_{2L})/K_{I}(D₃) ≠ nicht erfindungsgemäß | | |

## Patentansprüche

1. Verbindungen der allgemeinen Formel I 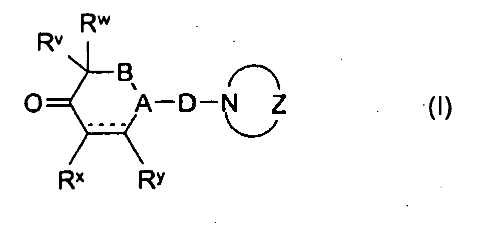 worin 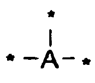 für eine Gruppe der Formel 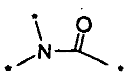 steht, worin D an das Stickstoffatom gebunden ist und worin das Kohlenstoffatom an die Variable B gebunden ist und * die Bindungsstellen angibt;
-B- für eine Bindung oder für 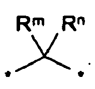 steht, worin R^{m} und Rⁿ unabhängig voneinander ausgewählt sind unter Wasserstoff, Halogen, gegebenen-falls substituiertem C₁-C₆-Alkyl, C₃-C₆-Cycloalkyl, C₂-C₆-Alkenyl, C₂-C₆-Alkinyl, C₁-C₆-Alkoxy, C₃-C₆-Cycloalkyloxy, C₃-C₆-Cycloalkyl-C₁-C₄-alkyloxy und gegebenenfalls substituiertem Phenyl, und * die Bindungs-stellen angibt;
··· für eine Einfachbindung oder eine Doppelbindung steht;
R^{v}, R^{w} unabhängig voneinander Wasserstoff, Halogen, gegebenenfalls substituiertes C₁-C₆-Alkyl, C₁-C₆-Alkoxy, C₂-C₆-Alkenyl, C₂-C₆-Alkinyl, C₃-C₆- Cycloalkyloxy, C₃-C₆-Cycloalkyl-C₁-C₄-alkyloxy oder C₃-C₆-Cycloalkyl bedeuten;
R^{x}, R^{y} gemeinsam mit den C-Atomen, an die sie gebunden sind, für einen kondensierten Phenylring oder ein kondensierten 5- oder 6-gliedrigen, aromatischen Heterocyclus stehen, der 1, 2, 3 oder 4 Heteroatome aufweist, die ausgewählt sind unter N, O und S, wobei der kondensierte Phenylring sowie der kondensierte aromatische Heterocyclus 1, 2 oder 3 Substituenten aufweisen können, die ausgewählt sind unter C₁-C₄-Alkyl, C₁-C₄-Hydroxyalkyl, C₁-C₄-Alkoxy-C₁-C₄-alkyl, CN, OR¹, NR²R³, NO₂, SR⁴, SO₂R⁴, SO₂NR²R³, CONR²R³, COOR⁵, COR⁶, C₁-C₂-Fluoralkyl, C₁-C₂-Fluoralkoxy. C₂-C₈-Alkenyl, C₂-C₆-Alkinyl, C₂-C₆-Alkenyloxy, C₂-C₆-Alkinyloxy, C₃-C₆-Cycloalkyl und Halogen, worin
R¹, R², R³, R⁴, R⁵ und R⁶ unabhängig voneinander für H, gegebenenfalls substituiertes C₁-C₆-Alkyl oder gegebenenfalls substituiertes Phenyl, stehen, wobei R³ auch eine Gruppe COR⁷ bedeuten kann, wobei R⁷ für Wasserstoff, gegebenenfalls substituiertes C₁-C₄-Alkyl oder gegebenen-falls substituiertes Phenyl stehen, wobei R² mit R³ auch gemeinsam einen 5- oder 6-gliedrigen, gesättigten oder ungesättigten Carbocyclus bilden kann, der ein Heteroatom, ausgewählt unter O, S und NR⁸ als Ringglied aufweisen kann, wobei R⁸ für Wasserstoff oder C₁-C₄-Alkyl steht,
D eine Gruppe (CH₂)ₖ oder eine Gruppe C(O)(CH₂)_{I} bedeutet, worin k für 3, 4, 5 oder 6 und I für 2, 3, 4 oder 5 stehen; 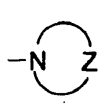
für einen Rest der Formel 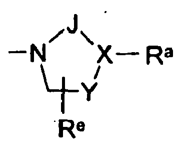 steht, worin
J für CH₂, CH₂-CH₂ oder CH₂-CH₂-CH₂ steht;
X für CH oder N und
Y für CH₂, CH₂-CH₂ oder CH₂-CH₂-CH₂ stehen oder Y-X gemeinsam CH=C oder CH₂-CH=C bedeuten;
R^{e} Wasserstoff oder C₁-C₄-Alkyl bedeutet, und
R^{a} für eine Gruppe E-Ar steht, worin E für eine Bindung oder für lineares oder verzweigtes Alkylen mit 1 bis 4 C-Atomen und insbesondere für (CH₂)ₚ steht, worin p für 0, 1, 2, 3 oder 4 steht, und Ar ausgewählt ist unter Phenyl, Naphthyl und 5- oder 6-gliederigem Heteroaryl, das ein, zwei oder drei Heteroatome ausgewählt unter S, O und N als Ringglieder aufweist und das gegebenenfalls 1, 2 oder 3 Substituenten R^{b} aufweisen kann, wobei R^{b} unabhängig voneinander ausgewählt sind unter gegebenenfalls substituiertem C₁-C₆-Alkyl, C₂-C₆-Alkenyl, C₂-C₆-Alkinyl, C₃-C₆-Cycloalkyl, C₄-C₁₀-Bicycloalkyl und C₆-C₁₀-Tricycloalkyl, wobei die drei letztgenannten Gruppen gegebenenfalls durch Halogen oder C₁-C₄-Alkyl substituiert sein können, Halogen, CN, OR¹, NR²R³, NO₂, SR⁴, SO₂R⁵, CONR²R³, SO₂NR²R³, COOR⁵, COR⁶, O-COR⁶, 5- oder 6-gliedriges Heterocyclyl mit 1, 2 oder 3 Heteroatomen, ausgewählt unter O, S und N, und Phenyl, wobei Phenyl und Heterocyclyl in den zwei zuletzt genannten Substituenten R^{b} gegebenenfalls ein oder zwei Substituenten tragen, die unabhängig voneinander ausgewählt sind unter C₁-C₄-Alkyl, C₁-C₄-Alkoxy, NR²R³, CN, C₁-C₂-Fluoralkyl und Halogen, und wobei 2 an benachbarte C-Atome des aromatischen Rests gebundene Substituenten R^{b} gemeinsam für C₃-oder C₄-Alkylen stehen können oder gemeinsam mit den C-Atomen, an die sie gebunden sind, für einen ankondensierten, ungesättigten 5 oder 6-gliedrigen Carbocyclus oder für einen 5- oder 6-gliedrigen Heterocyclus mit 1 oder 2 Stickstoffatomen als Ringglieder stehen können;
sowie
die physiologisch akzeptablen Säureadditionssalze dieser Verbindungen sowie die Tautomere der Formel I' 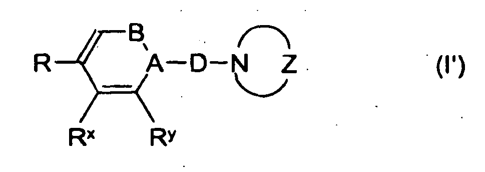 worin R für Halogen, eine Gruppe O-R', worin R¹ die zuvor genannten Bedeutungen aufweist, oder für eine Gruppe O-C(O)R⁹ steht, wobei R⁹ für Wasserstoff, gegebenenfalls substituiertes C₁-C₆-Alkyl, Benzyl oder Phenyl steht, wobei die zwei letztgenannten Reste gegebenenfalls durch ein oder zwei Reste substituiert ist, die unabhängig voneinander ausgewählt sind unter C₁-C₄-Alkyl, OH, C₁-C₄-Alkoxy, NR²R³, CN, C₁-C₂-Fluoralkyl oder Halogen, und die physiologisch akzeptablen Säureadditionssalze der Tautomere I',
wobei der Ausdruck "gegebenenfalls substituiertes Alkyl" in den Restedefinitionen von B, R^{v}, R^{w}, R¹, R², R³, R⁴, R⁵, R⁶, R⁷, R⁹, R^{b} und in der Restedefinition von "gegebenenfalls substituiertem Phenyl" für Alkyl steht, das teilweise oder vollständig durch Halogen substituiert sein kann und das einen oder mehrere von Halogen verschiedene Substituenten tragen kann, die ausgewählt sind unter CN, C₃-C₇-Cycloalkyl, C₃-C₇-Heterocycloalkyl, gegebenenfalls substituiertem Phenyl, OR¹¹, COOR¹¹ NR¹²R¹³, SO₂NR¹²R¹³, CONR¹²R¹³, O-CONR¹²R¹³, S-R¹⁴, SOR¹⁵, SO₂R¹⁵, OCOR¹⁶ und COR¹⁶, wobei R¹¹ die für R¹ angegebene Bedeutung, R¹² die für R² angegebene Bedeutung, R¹³ die für R³ angegebene Bedeutung, R¹⁴ die für R⁴ angegebene Bedeutung. R¹⁵ die für R⁵ angegebene Bedeutung und R¹⁶ die für R⁶ angegebene Bedeutung aufweisen, und
der Ausdruck "gegebenenfalls substituiertes Phenyl" in den Restedefinitionen von B, R¹, R², R³, R⁴, R⁵, R⁶, R⁷ und in der Restedefinition von "gegebenenfalls substituiertem Alkyl" für Phenyl steht, das gegebenenfalls einen oder mehrere Substituenten aufweist, die ausgewählt sind unter Halogen, Nitro, Cyano, gegebenenfalls substituiertem C₁-C₄-Alkyl, C₂-C₆-Alkenyl, C₂-C₆-Alkinyl, C₃-C₆-Cycloalkyl, C₁-C₄-Halogenalkyl, C₁-C₄-Alkoxy, OR²¹, COOR²¹, NR²²R²³, SO₂NR²²R²³, CONR²²R²³, O-CONR²²R²³, S-R²⁴, SOR²⁵, SO₂R²⁵, OCOR²⁶ und COR²⁶, wobei R²¹ die für R¹ angegebene Bedeutung, R²² die für R² angegebene Bedeutung, R²³ die für R³ angegebene Bedeutung, R²⁴ die für R⁴ angegebene Bedeutung, R²⁵ die für R⁵ angegebene Bedeutung und R²⁶ die für R⁶ angegebene Bedeutung aufweisen.

2. Verbindungen nach Anspruch 1, worin B für CH₂ steht.

3. Verbindungen nach einem der vorhergehenden Ansprüche, worin J für CH₂-CH₂ und Y für CH₂ stehen.

4. Verbindungen nach einem der vorhergehenden Ansprüche, worin X für N steht.

5. Verbindungen nach einem der vorhergehenden Ansprüche, worin E für eine Bindung steht.

6. Verbindungen nach Anspruch 5, worin Ar für Phenyl, Pyridyl, Pyrimidinyl oder s-Triazinyl steht, die 1, 2 oder 3 der vorgenannten Reste R^{b} aufweisen.

7. Verbindungen nach einem der Ansprüche 1 bis 4, worin E für CH₂ steht.

8. Verbindungen nach Anspruch 7, worin Ar für Phenyl, Naphthyl, Pyridyl, Pyridinyl, Pyrazinyl, Pyridazinyl, Thienyl, Furyl, Pyrrolyl, Pyrazolyl, Imidazolyl, Oxazolyl, Isoxazolyl, Thiazolyl, Isothiazolyl, 1-Oxa-3,4-diazolyl oder 1-Thia-3,4-diazolyl steht, die unsubstituiert sind oder 1, 2 oder 3 der obengenannten Reste R^{b} aufweisen können.

9. Verbindungen der allgemeinen Formel 1-Aa 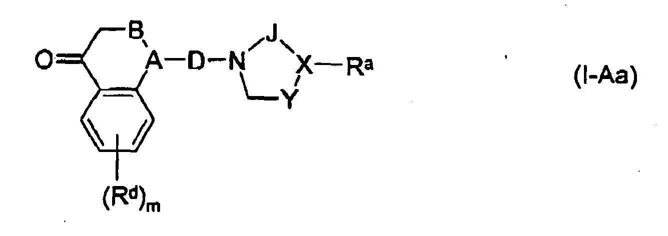
worin R^{a}, A, B und D die in Anspruch 1 angegebenen Bedeutungen aufweisen;
m für 0, 1, 2 oder 3 steht;
R^{d} unabhängig voneinander C₁-C₄-Alkyl, C₁-C₄-Hydroxyalkyl, C₁-C₄-Alkoxy-C₁-C₄-alkyl, CN, OR¹, NR²R³, NO₂, SR⁴, SO₂R⁴, SO₂NR²R³, CONR²R³, COOR⁶, COR⁶, C₁-C₂-Fluoralkyl, C₁-C₂-Fluoralkoxy, C₂-C₆-Alkenyl, C₂-C₆-Alkinyl, C₂-C₆-Alkenyloxy, C₂-C₆-Alkinyloxy, C₃-C₆-Cycloalkyl, C₃-C₆-Cycloalkyl oder Halogen bedeutet, wobei R¹, R², R³, R⁴, R⁶ und R⁶ die in Anspruch 1 angegebenen Bedeutungen aufweisen;
J für CH₂, CH₂-CH₂ oder CH₂-CH₂-CH₂ steht;
X für CH oder N und
Y für CH₂, CH₂-CH₂ oder CH₂-CH₂-CH₂ stehen oder Y-X gemeinsam CH=C oder CH₂-CH=C bedeuten;
die physiologisch akzeptablen Säureadditionssalze dieser Verbindungen sowie die Tautomere der Formel I-A'a 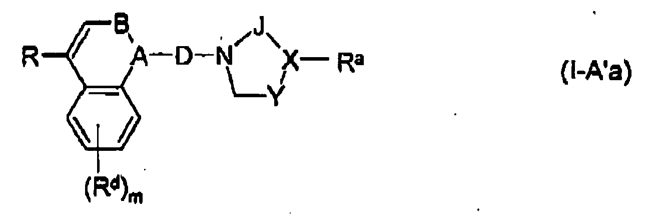 worin R die in Anspruch 1 angegebenen Bedeutungen aufweist und die physiologisch akzeptablen Säureadditionssalze der Tautomere I-A'a.

10. Verbindungen nach Anspruch 9, worin J für CH₂-CH₂ und Y für CH₂ stehen.

11. Verbindungen nach einem der Ansprüche 9 oder 10, worin X für N steht.

12. Verbindungen nach einem der Ansprüche 9 bis 11. worin E für eine Bindung steht.

13. Verbindungen nach Anspruch 12, worin Ar für Phenyl, Pyridyl, Pyrimidinyl oder s-Triazinyl steht, die 1, 2 oder 3 der vorgenannten Reste R^{b} aufweisen.

14. Verbindungen nach Anspruch 9, worin E für CH₂ steht.

15. Verbindungen nach Anspruch 14, worin Ar für Phenyl, Naphthyl, Pyridyl, Pyridinyl, Pyrazinyl, Pyridazinyl, Thienyl, Furyl, Pyrrolyl, Pyrazolyl, Imidazolyl, Oxazolyl, Isoxazolyl, Thiazolyl, Isothiazolyl, 1-Oxa-3,4-diazolyl oder 1-Thia-3,4-diazolyl steht, die unsubstituiert sind oder 1, 2 oder 3 der obengenannten Reste R^{b} aufweisen können.

16. Pharmazeutisches Mittel, enthaltend wenigstens einen Wirkstoff, der ausgewählt ist unter Verbindungen der Formeln I, den Tautomeren der Formel I', den physiologisch verträglichen Säureadditionssalzen der Verbindungen I und den physiologisch verträglichen Säureadditionssalzen der Tautomere der Formel I' gemäß einem der Ansprüche 1 bis 15, gegebenenfalls zusammen mit physiologisch akzeptablen Trägern und/oder Hilfsstoffen.

17. Verwendung von Wirkstoffen, die ausgewählt sind unter Verbindungen der Forme in I, den Tautomeren der Formel I', den physiologisch verträglichen Säureadditionssalzen der Verbindungen I und den physiologisch verträglichen Säureadditionssalzen der Tautomere der Formel I' gemäß einem der Ansprüche 1 bis 15, zur Herstellung eines pharmazeutischen Mittels zur Behandlung von Erkrankungen, die auf die Beeinflussung durch Dopamin-D₃-Rezeptorantagonisten bzw. -agonisten ansprechen.

18. Verwendung nach Anspruch 17 zur Behandlung von Erkrankungen des zentralen Nervensystems.

19. Verwendung nach Anspruch 17 zur Behandlung von Nierenfunktionsstörungen.

## Claims

1. Compounds of the general formula I 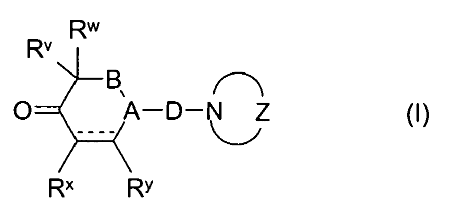 where 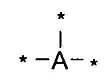 is a group of the formula 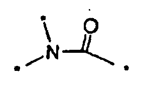 where D is bonded to the nitrogen atom and where the carbon atom is bonded to the variable B and * denotes the bonding sites;
-B- is a bond or 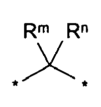 where R^{m} and Rⁿ are each independently selected from hydrogen, halogen, optionally substituted C₁-C₆-alkyl, C₃-C₆-cycloalkyl, C₂-C₆-alkenyl, C₂-C₆-alkynyl, C₁-C₆-alkoxy, C₃-C₆-cycloalkyloxy, C₃-C₆-cycloalkyl-C₁-C₄-alkyloxy and optionally substituted phenyl, and * denotes the bonding sites;
R^{v} R^{w} are each independently hydrogen, halogen, optionally substituted C₁-C₆-alkyl, C₁-C₆-alkoxy, C₂-C₆-alkenyl, C₂-C₆-alkynyl, C₃-C₆-cycloalkyloxy, C₃-C₆-cycloalkyl-C₁-C₄-alkyloxy or C₃-C₆-cycloalkyl;
R^{x}, R^{y}, together with the carbon atoms to which they are bonded, are a fused phenyl ring or a fused 5- or 6-membered aromatic heterocycle which has 1, 2, 3 or 4 heteroatoms which are selected from N, O and S, where the fused phenyl ring and the fused aromatic heterocycle may have 1, 2 or 3 substituents which are selected from C₁-C₄-alkyl, C₁-C₄-hydroxyalkyl, C₁-C₄-alkoxy-C₁-C₄-alkyl, CN, OR¹, NR²R³, NO₂, SR⁴, SO₂R⁴, SO₂NR²R³, CONR²R³, COOR⁵, COR⁶, C₁-C₂-fluoroalkyl, C₁-C₂-fluoroalkoxy, C₂-C₆-alkenyl, C₂-C₆-alkynyl, C₂-C₆-alkenyloxy, C₂-C₆-alkynyloxy, C₃-C₆-cycloalkyl and halogen; where
R¹, R², R³, R⁴, R⁵ and R⁶ are each independently H, optionally substituted C₁-C₆-alkyl or optionally substituted phenyl, where R³ may also be a COR⁷ group where R⁷ is hydrogen, optionally substituted C₁-C₄-alkyl or optionally substituted phenyl, where R² with R³ may also together form a 5- or 6-membered, saturated or unsaturated carbocycle which may have a heteroatom selected from O, S and NR⁸ as a ring member, where R⁸ is hydrogen or C₁-C₄-alkyl,
D is a (CH₂)ₖ group or a C(O)(CH₂)₁ group, where k is 3, 4, 5 or 6 and 1 is 2, 3, 4 or 5; 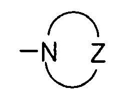 is a radical of the formula 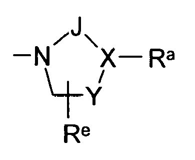
J is CH₂, CH₂-CH₂ or CH₂-CH₂-CH₂;
X is CH or N and
Y is CH₂, CH₂-CH₂ or CH₂-CH₂-CH₂, or Y-X together is CH=C or CH₂-CH=C;
R^{e} is hydrogen or C₁-C₄-alkyl, and
R^{a} is an E-Ar group wherein E is a bond or linear or branched alkylene having from 1 to 4 carbon atoms and in particular (CH₂)ₚ where p is 0, 1, 2, 3 or 4, and Ar is selected from phenyl, naphthyl and 5- or 6-membered heteroaryl which has one, two or three heteroatoms selected from S, O and N as ring members and which may optionally have 1, 2 or 3 substituents R^{b} where R^{b} is independently selected from optionally substituted C₁-C₆-alkyl, C₂-C₆-alkenyl, C₂-C₆-alkynyl, C₃-C₆-cycloalkyl, C₄-C₁₀-bicycloalkyl and C₆-C₁₀-tricycloalkyl, where the last three groups may optionally be substituted by halogen or C₁-C₄-alkyl, halogen, CN, OR¹, NR²R³, NO₂, SR⁴, SO₂R⁵, CONR²R³, SO₂NR²R³, COOR⁵, COR⁶, O-COR⁶, 5- or 6-membered heterocyclyl having 1, 2 or 3 heteroatoms selected from O, S and N, and phenyl, where phenyl and heterocyclyl in the last two substituents R^{b} may optionally bear one or two substituents which are each independently selected from C₁-C₄-alkyl, C₁-C₄-alkoxy, NR²R³, CN, C₁-C₂-fluoroalkyl and halogen, and where 2 substituents R^{b} bonded to adjacent carbon atoms of the aromatic radical may together be C₃- or C₄-alkylene, or, together with the carbon atoms to which they are bonded, may be a fused-on, unsaturated 5- or 6-membered carbocycle or a 5- or 6-membered heterocycle having 1 or 2 nitrogen atoms as ring members;
and
the physiologically acceptable acid addition salts of this compound and the tautomer of the formula I' 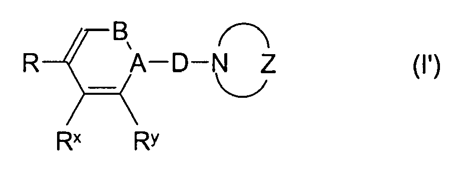 where R is halogen, an O-R¹ group where R¹ is as defined above, or an O-C(O)R⁹ group where R⁹ is hydrogen, optionally substituted C₁-C₆-alkyl, benzyl or phenyl, where the last two radicals are optionally substituted by one or two radicals which are each independently selected from C₁-C₄-alkyl, OH, C₁-C₄-alkoxy, NR²R³, CN, C₁-C₂-fluoroalkyl or halogen, and the physiologically acceptable acid addition salts of the tautomer I',
where the expression "optionally substituted alkyl" in the radical definitions of B, R^{v}, R^{w}, R¹, R², R³ R⁴, R⁵, R⁶, R⁷, R⁹, R^{b} and in the radical definition of "optionally substituted phenyl" represents alkyl which may be partly or fully substituted by halogen and which may bear one or more substituents other than halogen which are selected from CN, C₃-C₇-cycloalkyl, C₃-C₇-heterocycloalkyl, optionally substituted phenyl, OR¹¹, COOR¹¹, NR¹²R¹³, SO₂NR¹²R¹³, CONR¹²R¹³, O-CONR¹²R¹³, S-R¹⁴, SOR¹⁵, SO₂R¹⁵, OCOR¹⁶ and COR¹⁶, where R¹¹ is as defined for R¹, R¹² is as defined for R², R¹³ is as defined for R³, R¹⁴ is as defined for R⁴, R¹⁵ is as defined for R⁵ and R¹⁶ is as defined for R⁶, and
the expression "optionally substituted phenyl" in the radical definitions of B, R¹, R², R³, R⁴, R⁵, R⁶, R⁷ and in the radical definition of "optionally substituted alkyl" represents phenyl which optionally has one or more substituents which are selected from halogen, nitro, cyano, optionally substituted C₁-C₄-alkyl, C₂-C₆-alkenyl, C₂-C₆-alkynyl, C₃-C₆-cycloalkyl, C₁-C₄-haloalkyl, C₁-C₄-alkoxy, OR²¹, COOR²¹, NR²²R²³, SO₂NR²²R²³, CONR²²R²³, O-CONR²²R²³, S-R²⁴, SOR²⁵, SO₂R²⁵, OCOR²⁶ and COR²⁶, where R²¹ is as defined for R¹, R²² is as defined for R², R²³ is as defined for R³, R²⁴ is as defined for R⁴, R²⁵ is as defined for R⁵ and R²⁶ is as defined for R⁶.

2. Compounds according to Claim 1, where B is CH₂.

3. Compounds according to any of the preceding claims, where J is CH₂-CH₂ and Y is CH₂.

4. Compounds according to any of the preceding claims, where X is N.

5. Compounds according to any of the preceding claims, where E is a bond.

6. Compounds according to Claim 5, where Ar is phenyl, pyridyl, pyrimidinyl or s-triazinyl, each of which has 1, 2 or 3 of the aforementioned R^{b} radicals.

7. Compounds according to any of Claims 1 to 4, where E is CH₂.

8. Compounds according to Claim 7, where Ar is phenyl, naphthyl, pyridyl, pyridinyl, pyrazinyl, pyridazinyl, thienyl, furyl, pyrrolyl, pyrazolyl, imidazolyl, oxazolyl, isoxazolyl, thiazolyl, isothiazolyl, 1-oxa-3,4-diazolyl or 1-thia-3,4-diazolyl, each of which is unsubstituted or may have 1, 2 or 3 of the abovementioned R^{b} radicals.

9. Compounds of the general formula I-Aa 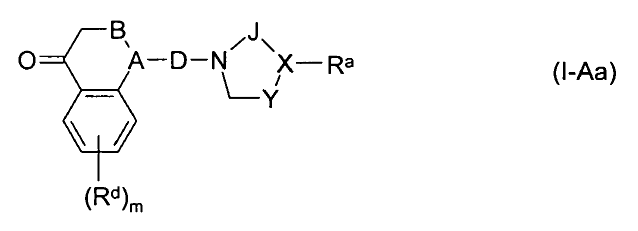
where R^{a}, A, B and D are each as defined in Claim 1;
m is 0, 1, 2 or 3;
R^{d} are each independently C₁-C₄-alkyl, C₁-C₄-hydroxyalkyl, C₁-C₄-alkoxy-C₁-C₄-alkyl, CN, OR¹, NR²R³, NO₂, SR⁴, SO₂R⁴, SO₂NR²R³, CONR²R³, COOR⁵, COR⁶, C₁-C₂-fluoroalkyl, C₁-C₂-fluoroalkoxy, C₂-C₆-alkenyl, C₂-C₆-alkynyl, C₂-C₆-alkenyloxy, C₂-C₆-alkynyloxy, C₃-C₆-cycloalkyl, C₃-C₆-cycloalkyl or halogen, where R¹, R², R³, R⁴, R⁵ and R⁶ are each as defined in claim 1;
J is CH₂, CH₂-CH₂ or CH₂-CH₂-CH₂;
X is CH or N and
Y is CH₂, CH₂-CH₂ or CH₂-CH₂-CH₂, or Y-X together is CH=C or CH₂-CH=C;
the physiologically acceptable acid addition salts of this compound and the tautomer of the formula I-A'a 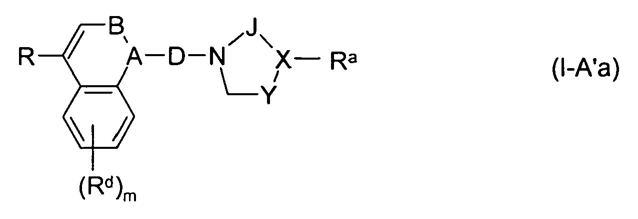 where R is as defined in Claim 1 and the physiologically acceptable acid addition salts of the tautomer I-A'a.

10. Compounds according to Claim 9, where J is CH₂-CH₂ and Y is CH₂.

11. Compounds according to either of Claims 9 and 10, where X is N.

12. Compounds according to any of Claims 9 to 11, where E is a bond.

13. Compounds according to Claim 12, where Ar is phenyl, pyridyl, pyrimidinyl or s-triazinyl, each of which has 1, 2 or 3 of the aforementioned R^{b} radicals.

14. Compounds according to Claim 9, where E is CH₂.

15. Compounds according to Claim 14, where Ar is phenyl, naphthyl, pyridyl, pyridinyl, pyrazinyl, pyridazinyl, thienyl, furyl, pyrrolyl, pyrazolyl, imidazolyl, oxazolyl, isoxazolyl, thiazolyl, isothiazolyl, 1-oxa-3,4-diazolyl or 1-thia-3,4-diazolyl, each of which is unsubstituted or may have 1, 2 or 3 of the abovementioned R^{b} radicals.

16. Pharmaceutical composition comprising at least one active ingredient which is selected from compounds of the formula I, the tautomers of the formula I', the physiologically tolerated acid addition salts of the compounds I and the physiologically tolerated acid addition salts of the tautomers of the formula I' according to any of Claims 1 to 15, optionally together with physiologically acceptable carriers and/or excipients.

17. Use of active ingredients which are selected from compounds of the formula I, the tautomers of the formula I', the physiologically tolerated acid addition salts of the compounds I and the physiologically tolerated acid addition salts of the tautomers of the formula I' according to any of Claims 1 to 15 for producing a pharmaceutical composition for treating diseases which respond to the influence of dopamine D₃ receptor antagonists or agonists.

18. Use according to Claim 17 for treating diseases of the central nervous system.

19. Use according to Claim 17 for treating kidney function disorders.

## Revendications

1. Composés de formule générale 1 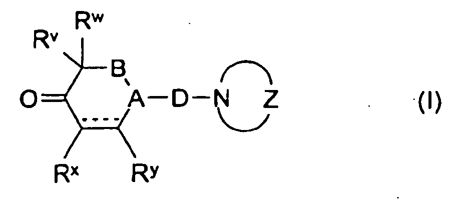 où 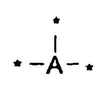 représente un groupe de formule 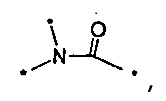 dans laquelle D est lié à l'atome d'azote et dans laquelle l'atome de carbone est lié à la variable B et * indique les sites de liaison ;
-B- représente une liaison ou 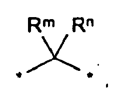 où R^{m} et Rⁿ sont choisis, indépendamment l'un de l'autre, parmi hydrogène, halogène, C₁-C₆-alkyle le cas échéant substitué, C₃-C₆-cycloalkyle, C₂-C₆-alcényle, C₂-C₆-alcynyle, C₁-C₆-alcoxy, C₃-C₆-cycloalkyloxy, C₃-C₆-cycloalkyl-C₁-C₄-alkyloxy, et phényle le cas échéant substitué, et * indique les sites de liaison ;
··· représente une simple ou une double liaison
R^{v}, R^{w} signifient, indépendamment l'un de l'autre, hydrogène, halogène, C₁-C₆-alkyle le cas échéant substitué, C₁-C₆-alcoxy, C₂-C₆-alcényle, C₂-C₆-alcynyle, C₃-C₆-cycloalkyloxy, C₃-C₆-cycloalkyl-C₁-C₄-alkyloxy ou C₃-C₆-cycloalkyle ;
R^{x}, R^{y} représentent, ensemble avec les atomes de C auxquels ils sont liés, un cycle phényle condensé ou un hétérocycle aromatique condensé de 5 ou 6 chaînons, qui présente 1, 2, 3 ou 4 hétéroatomes, qui sont choisis parmi N, O et S, le cycle phényle condensé ainsi que l'hétérocycle aromatique condensé pouvant présenter 1, 2 ou 3 substituants, qui sont choisis parmi C₁-C₄-alkyle, C₁-C₄-hydroxyalkyle, C₁-C₄-alcoxy-C₁-C₄-alkyle, CN, OR¹, NR²R³, NO₂, SR⁴, SO₂R⁴, SO₂NR²R³, CONR²R₃, COOR⁵, COR⁶, C₁-C₂-fluoroalkyle, C₁-C₂-fluoroalcoxy, C₂-C₆-alcényle, C₂-C₆-alcynyle, C₂-C₆-alcényloxy, C₂-C₆-alcynyloxy, C₃-C₆-cycloalkyle et halogène, où R¹, R², R³, R⁴, R⁵ et R⁶, indépendamment l'un de l'autre, représentent H, C₁-C₆-alkyle le cas échéant substitué ou phényle le cas échéant substitué, R³ pouvant également signifier un groupe COR⁷, R⁷ représentant hydrogène, C₁-C₄-alkyle le cas échéant substitué ou phényle le cas échéant substitué, où R² peut également former ensemble avec R³ un cycle carboné de 5 ou 6 chaînons, saturé ou insaturé, qui peut présenter un hétéroatome, choisi parmi O, S et NR⁸ comme élément de cycle, où R⁸ représente hydrogène ou C₁-C₄-alkyle,
D représente un groupe (CH₂)ₖ ou un groupe C(O)(CH₂)₁, où k représente 3, 4, 5 ou 6 et 1 représente 2, 3, 4 ou 5 ; 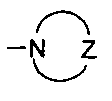 représente un radical de formule 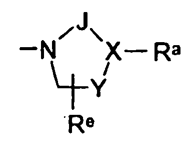 où
J représente CH₂, CH₂-CH₂ ou CH₂-CH₂-CH₂ ;
X représente CH ou N et
Y représente CH₂, CH₂-CH₂ ou CH₂-CH₂-CH₂ ou Y-X signifient, ensemble, CH=C ou CH₂-CH=C ;
R^{e} signifie hydrogène ou C₁-C₄-alkyle, et
R^{a} représente un groupe E-Ar, où E représente une liaison ou un groupe alkylène linéaire ou ramifié, comprenant 1 à 4 atomes de carbone et en particulier (CH₂)ₚ, où p représente 0, 1, 2, 3 ou 4, et Ar est choisi parmi phényle, naphtyle et hétéroaryle à 5 ou 6 chaînons, qui présente un, deux ou trois hétéroatomes choisis parmi S, O et N comme éléments de cycle et qui peut le cas échéant présenter 1, 2 ou 3 substituants R^{b}, où les substituants R^{b} sont choisis, indépendamment l'un de l'autre, parmi C₁-C₆-alkyle le cas échéant substitué, C₂-C₆-alcényle, C₂-C₆-alcynyle, C₃-C₆-cycloalkyle, C₄-C₁₀-bicycloalkyle et C₆-C₁₀-tricycloalkyle, où les trois derniers groupes mentionnés peuvent le cas échéant être substitués par halogène ou C₁-C₄-alkyle, halogène, CN, OR¹, NR²R³, NO₂, SR⁴, SO₂R⁵, CONR²R³, SO₂NR²R³, COOR⁵, COR⁶, O-COR⁶, hétérocyclyle de 5 ou 6 chaînons comprenant 1, 2 ou 3 hétéroatomes, choisis parmi O, S et N, et phényle, où phényle et hétérocyclyle dans les deux derniers substituants R^{b} mentionnés portent le cas échéant un ou deux substituants, qui sont choisis, indépendamment l'un de l'autre, parmi C₁-C₄-alkyle, C₁-C₄-alcoxy, NR²R³, CN, C₁-C₂-fluoroalkyle et halogène, et où 2 substituants R^{b} liés à des atomes de carbone adjacents du radical aromatique peuvent représenter ensemble alkylène en C₃ ou C₄ ou peuvent représenter, ensemble avec les atomes de carbone auxquels ils sont liés, un cycle carboné condensé, insaturé de 5 ou 6 chaînons ou un hétérocycle de 5 ou 6 chaînons comprenant 1 à 2 atomes d'azote comme éléments de cycle ;
ainsi que
les sels d'addition d'acide physiologiquement acceptables de ces composés, ainsi que les tautomères de formule I' 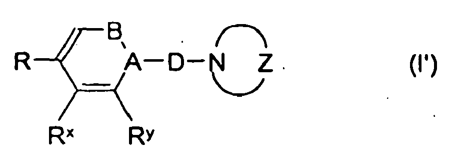 où R représente halogène, un groupe O-R¹, où R¹ présente les significations susmentionnées, ou un groupe OC(O)R⁹, où R⁹ représente hydrogène, C₁-C₆-alkyle le cas échéant substitué, benzyle ou phényle, les deux derniers radicaux mentionnés pouvant le cas échéant être substitués par un ou deux radicaux, qui sont choisis, indépendamment l'un de l'autre, parmi C₁-C₄-alkyle, OH, C₁-C₄-alcoxy, NR²R³, CN, C₁-C₂-fluoroalkyle ou halogène, et les sels d'addition d'acide physiologiquement acceptables des tautomères I', l'expression "alkyle le cas échéant substitué" dans les définitions des radicaux de B, R^{v}, R^{w}, R¹, R², R³, R⁴, R⁵, R⁶, R⁷, R⁹, R^{b} et dans les définitions des radicaux de "phényle le cas échéant substitué" désignant alkyle, qui peut être partiellement ou totalement substitué par halogène et qui peut porter un ou plusieurs substituants différents d'halogène, qui sont choisis parmi CN, C₃-C₇-cycloalkyle, C₃-C₇-hétérocycloalkyle, phényle le cas échéant substitué, OR¹¹, COOR¹¹, NR¹²R¹³, SO₂NR¹²R¹³, CONR¹²R¹³, O-CONR¹²R¹³, S-R¹⁴, SOR¹⁵, SO₂R¹⁵, OCOR¹⁶ et COR¹⁶, où R¹¹ a la signification indiquée pour R¹, R¹² a la signification indiquée pour R², R¹³ a la signification indiquée pour R³, R¹⁴ a la signification indiquée pour R⁴, R¹⁵ a la signification indiquée pour R⁵ et R¹⁶ a la signification indiquée pour R⁶, et l'expression "phényle le cas échéant substitué" dans les définitions des radicaux de B, R¹, R², R³, R⁴, R⁵, R⁶, R⁷ et dans les définitions des radicaux de "alkyle le cas échéant substitué" désignant phényle, qui présente le cas échéant un ou plusieurs substituants, qui sont choisis parmi halogène, nitro, cyano, C₁-C₄-alkyle le cas échéant substitué, C₂-C₆-alcényle, C₂-C₆-alcynyle, C₃-C₆-cycloalkyle, C₁-C₄-halogénoalkyle, C₁-C₄-alcoxy, OR²¹, COOR²¹, NR²²R²³, SO₂NR²²R²³, CONR²²R²³, O-CONR²²R²³, S-R²⁴, SOR²⁵, SO₂R²⁵, OCOR²⁶ et COR²⁶, où R²¹ a la signification indiquée pour R¹, R²² a la signification indiquée pour R², R²³ a la signification indiquée pour R³, R²⁴ a la signification indiquée pour R⁴, R²⁵ a la signification indiquée pour R⁵ et R²⁶ a la signification indiquée pour R⁶.

2. Composés selon la revendication 1, où B représente CH₂.

3. Composés selon l'une quelconque des revendications précédentes, où J représente CH₂-CH₂ et Y représente CH₂.

4. Composés selon l'une quelconque des revendications précédentes, où X représente N.

5. Composés selon l'une quelconque des revendications précédentes, où E représente une liaison.

6. Composés selon la revendication 5, où Ar représente phényle, pyridyle, pyrimidinyle ou s-triazinyle, qui présentent 1, 2 ou 3 des radicaux R^{b} susmentionnés.

7. Composés selon l'une quelconque des revendications 1 à 4, où E représente CH₂.

8. Composés selon la revendication 7, où Ar représente phényle, naphtyle, pyridyle, pyridinyle, pyrazinyle, pyridazinyle, thiényle, furyle, pyrrolyle, pyrazolyle, imidazolyle, oxazolyle, isoxazolyle, thiazolyle, isothiazolyle, 1-oxa-3,4-diazolyle ou 1-thia-3,4-diazolyle, qui sont non substitués ou qui peuvent présenter 1, 2 ou 3 des radicaux R^{b} susmentionnés.

9. Composés de formule générale I-Aa 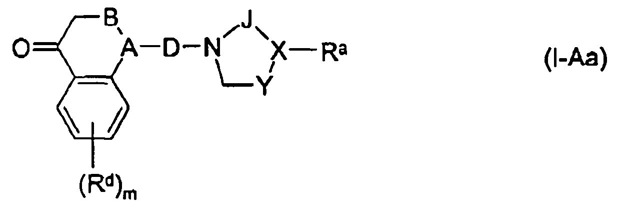 où
R^{a}, A, B et D présentent les significations indiquées dans la revendication 1 ;
m représente 0, 1, 2 ou 3 ;
les radicaux R^{d} signifient, indépendamment l'un de l'autre, C₁-C₄-alkyle, C₁-C₄-hydroxyalkyle, C₁-C₄-alcoxy-C₁-C₄-alkyle, CN, OR¹, NR²R³, NO₂, SR⁴, SO₂R⁴, SO₂NR²R³, CONR²R³, COOR⁵, COR⁶, C₁-C₂-fluoroalkyle, C₁-C₂-fluoroalcoxy, C₂-C₆-alcényle, C₂-C₆-alcynyle, C₂-C₆-alcényloxy, C₂-C₆-alcynyloxy, C₃-C₆-cycloalkyle, C₃-C₆-cycloalkyle ou halogène, où R¹, R², R³, R⁴, R⁵ et R⁶ présentent les significations indiquées dans la revendication 1 ;
J représente CH₂, CH₂CH₂ ou CH₂-CH₂-CH₂ ;
X représente CH ou N et
Y représente CH₂, CH₂-CH₂ ou CH₂-CH₂-CH₂ ou Y-X signifient, ensemble, CH=C ou CH₂-CH=C ;
les sels d'addition d'acide physiologiquement acceptables de ces composés, ainsi que les tautomères de formule I-A'a 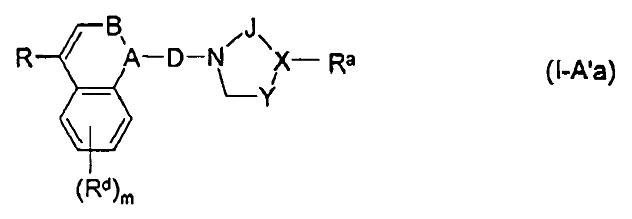 où
R présente les significations indiquées dans la revendication 1 et les sels d'addition d'acide physiologiquement acceptables des tautomères I-A'a.

10. Composés selon la revendication 9, où J représente CH₂-CH₂ et Y représente CH₂.

11. Composés selon l'une quelconque des revendications 9 ou 10, où X représente N.

12. Composés selon l'une quelconque des revendications 9 à 11, où E représente une liaison.

13. Composés selon la revendication 12, où Ar représente phényle, pyridyle, pyrimidinyle ou s-triazinyle, qui présentent 1, 2 ou 3 des radicaux R^{b} susmentionnés.

14. Composés selon la revendication 9, où E représente CH₂.

15. Composés selon la revendication 14, où Ar représente phényle, naphtyle, pyridyle, pyridinyle, pyrazinyle, pyridazinyle, thiényle, furyle, pyrrolyle, pyrazolyle, imidazolyle, oxazolyle, isoxazolyle, thiazolyle, isothiazolyle, 1-oxa-3,4-diazolyle ou 1-thia-3,4-diazolyle, qui sont non substitués ou qui peuvent présenter 1, 2 ou 3 radicaux R^{b} susmentionnés.

16. Agent pharmaceutique, contenant au moins une substance active qui est choisie parmi les composés de formule I, les tautomères de formule I', les sels d'addition d'acide physiologiquement acceptables des composés I et les sels d'addition d'acide physiologiquement acceptables des tautomères de formule I' selon l'une quelconque des revendications 1 à 15, le cas échéant avec des supports et/ou des adjuvants physiologiquement acceptables.

17. Utilisation de substances actives, qui sont choisies parmi les composés de formule I, les tautomères de formule I', les sels d'addition d'acide physiologiquement acceptables des composés I et les sels d'addition d'acide physiologiquement acceptables des tautomères de formule I' selon l'une quelconque des revendications 1 à 15, pour la préparation d'un agent pharmaceutique pour le traitement de maladies, qui réagissent à l'influence par les antagonistes ou les agonistes des récepteurs de la dopamine D₃.

18. Utilisation selon la revendication 17 pour le traitement de maladies du système nerveux central.

19. Utilisation selon la revendication 17 pour le traitement de troubles de la fonction rénale.
